# EUROPEAN PATENT SPECIFICATION

(11) **EP 3 142 506 B1**
(45) Date of publication and mention of the grant of the patent: **19.12.2018**
(21) Application number: 15792261.8
(22) Date of filing: 13.05.2015
(51) Int. Cl.: A24F 47/00, H04L 29/06, A61M 15/06

(54) **ELECTRONIC SMOKING DEVICE AND DATA EXCHANGE APPLICATIONS**
ELEKTRONISCHE RAUCHVORRICHTUNG UND DATENAUSTAUSCHANWENDUNGEN
DISPOSITIF ÉLECTRONIQUE POUR FUMEUR ET APPLICATIONS D'ÉCHANGE DE DONNÉES

(30) Priority: 13.05.2014 US 201461992674 P
(43) Date of publication of application: 22.03.2017
(73) Proprietor: Fontem Holdings 4 B.V., 1083 HN Amsterdam (NL)
(72) Inventor: ALARCON, Ramon, Los Gatos, California 95030 (US); RASMUSSEN, Dennis, Campbell, California 95008 (US)
(74) Representative: Gulde & Partner
(86) International application number: PCT/US2015/030633
(87) International publication number: WO 2015/175701

(56) References cited:
- US-A1- 2011 265 806
- US-A1- 2013 265 702
- US-A1- 2013 319 439
- US-A1- 2013 319 439
- US-A1- 2013 340 775
- BO LI ET AL: "i-Function of Electronic Cigarette: Building Social Network by Electronic Cigarette", INTERNET OF THINGS (ITHINGS/CPSCOM), 2011 INTERNATIONAL CONFERENCE ON AND 4TH INTERNATIONAL CONFERENCE ON CYBER, PHYSICAL AND SOCIAL COMPUTING, IEEE, 19 October 2011 (2011-10-19), pages 634-637, XP032107062, DOI: 10.1109/ITHINGS/CPSCOM.2011.124 ISBN: 978-1-4577-1976-9

## Description

### CROSS-REFERENCE TO PRIOR APPLICATIONS

This application claims priority to United States provisional patent application no. 61/992,674, filed on 13 May 2014.

### BACKGROUND OF THE DISCLOSURE

### 1. Field of the Disclosure

This disclosure is directed to an electronic smoking device, and particularly to an electronic smoking device and an associated pack with enhanced features and functionalities for use therewith.

### 2. Related Art

Electronic cigarettes are a popular alternative to traditional tobacco based cigarettes that must be burned in order to generate smoke for inhalation. Electronic cigarettes provide a vapor for inhalation, but do not contain certain byproducts of combustion that may be harmful to human health. However, electronic cigarettes are a relatively new invention and current systems do not deliver the same "quality" of experience as traditional cigarettes. For example, electronic cigarettes have relatively slow rate of vaporization and this tends to produce an inconsistent quality of vapor. This may be due to the use of a wick that transports liquid from a disposable cartridge to the vaporizing element. The "wicking" method of fluid transport is a relatively slow method and therefore limits the rate at which the user can smoke the cigarette. Additionally, the wick limits the ability to control and monitor the amount of nicotine delivered to the user. Finally, the wick construction is more difficult to assemble and automate manufacturing, has limited quality, and may be contaminated.

Additionally, the user interface of early generation electronic cigarettes do not provide clear and intuitive information to the user. For example, while traditional cigarettes provide a visual indication when the smoking product has been exhausted, electronic cigarettes do not provide a similar clear indication.

The publications US2011265806, US2013319439 and XP032107062 represent relevant prior art disclosing systems of electronic smoking devices exchanging usage information over the Internet, e.g. in social media.

Some users chose to smoke electronic cigarettes as part of a smoking cessation program. However, it is often difficult for the user to determine the exact amount of the product being consumed and thus difficult to measure the progress of such a cessation program. Accordingly, there is a need for an improved electronic cigarette.

### SUMMARY OF THE DISCLOSURE

According to an aspect of the disclosure, an electronic smoking system includes: an electronic smoking device; a first pack communicatively linked to the device, the first pack configured to hold the device; and a first computer communicatively linked to the pack and to at least one communication channel; wherein the communication channel is configured to share data with at least one of a social network, a vendor, a clinical monitoring program, or a data collection program.

According to another aspect of the disclosure, a method includes receiving data recorded or stored on at least one of a user's electronic smoking device or pack; using the data to determine a status of at least one of the electronic smoking device, the pack, or the user; and transmitting the status to at least one of the user or a third party recipient.

According to another aspect of the disclosure, a portable electronic device includes: communication circuitry; a storage component operable to store data related to at least one of a user, the user's electronic smoking device, or the user's pack; and control circuitry operable to: determine, based on the stored data, a status of at least one of the electronic smoking device, the pack, or the user; and instruct the communication circuitry to transmit the status to at least one of the user or a third party recipient.

According to another aspect of the disclosure, machine-readable media include machine-readable instructions recorded thereon for: receiving data recorded or stored on at least one of a user's electronic smoking device or pack; using the data to determine a status of at least one of the electronic smoking device, the pack, or the user; and transmitting the status to at least one of the user or a third party recipient.

Additional features, advantages, and embodiments of the disclosure may be set forth or apparent from consideration of the following detailed description, drawings, and claims. Moreover, it is to be understood that both the foregoing summary of the disclosure and the following detailed description are exemplary and intended to provide further explanation without limiting the scope of the disclosure as claimed.

### BRIEF DESCRIPTION OF THE DRAWINGS

The accompanying drawings, which are included to provide a further understanding of the disclosure, are incorporated in and constitute a part of this specification, illustrate embodiments of the disclosure and together with the detailed description serve to explain the principles of the disclosure. No attempt is made to show structural details of the disclosure in more detail than may be necessary for a fundamental understanding of the disclosure and the various ways in which it may be practiced. In the drawings:
FIG. 1A shows a structural overview of an electronic smoking device constructed according to the principles of the disclosure.
FIG. 1B shows a schematic overview of another aspect of the electronic smoking device constructed according to the principles of the disclosure.
FIG. 2A shows a cross-section view of an exemplary design of the electronic smoking devices shown in FIGS. 1A and 1B, constructed according to the principles of the disclosure.
FIG. 2B shows an exploded view of the electronic smoking device shown in FIG. 2A.
FIG. 3 shows a partial perspective view of an air flow path, a container, a housing and a micromesh screen of the electronic smoking device shown in FIG. 2A, constructed according to the principles of the disclosure.
FIG. 4 shows an enlarged view of the micromesh screen shown in FIG. 3, constructed according to the principles of the disclosure.
FIG. 5 shows a perspective view of a solid state heater of the electronic smoking device shown in FIG. 2A, constructed according to the principles of the disclosure.
FIG. 6 shows the solid state heater shown in FIG. 5 arranged in association with the micromesh screen shown in FIG. 4.
FIG. 7A shows a perspective view of a pack for the electronic smoking device, constructed according to the principles of the disclosure.
FIG. 7B shows a perspective view of another pack for electronic smoking device, constructed according to the principles of the disclosure.
FIG. 7C shows a bottom perspective view of the pack shown in FIG. 7.
FIG. 8 shows a schematic overview of the pack shown in FIG. 7, constructed according to the principles of the disclosure.
FIG. 9A shows a conceptual overview of a system for exchanging data over various communication channels using the pack shown in FIG. 7, constructed according to the principles of the disclosure.
FIG. 9B shows another conceptual overview of a system for exchanging data over various communication channels.
FIGS. 9C-9M show examples of components of a smart phone app for use in conjunction with the electronic smoking device.
FIGS. 10 and 11 show a schematic of a sensor for the electronic smoking device constructed according to the principles of the disclosure.
FIGS. 12 and 13 show a schematic of another sensor for the electronic smoking device constructed according to the principles of the disclosure.
FIGS. 14 and 15 show a schematic of yet another sensor for the electronic smoking device constructed according to the principles of the disclosure.
FIGS. 16, 17, 18, 19, 20 and 21 show flowcharts of various processes for carrying several advanced functionalities in an electronic smoking device according to the principles of the disclosure.

### DETAILED DESCRIPTION OF THE DISCLOSURE

The embodiments of the disclosure and the various features and advantageous details thereof are explained more fully with reference to the non-limiting embodiments and examples that are described and/or illustrated in the accompanying drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the disclosure. The examples used herein are intended merely to facilitate an understanding of ways in which the disclosure may be practiced and to further enable those of skill in the art to practice the embodiments of the disclosure. Accordingly, the examples and embodiments herein should not be construed as limiting the scope of the disclosure, which is defined solely by the appended claims and applicable law. Moreover, it is noted that like reference numerals represent similar parts throughout the several views of the drawings.

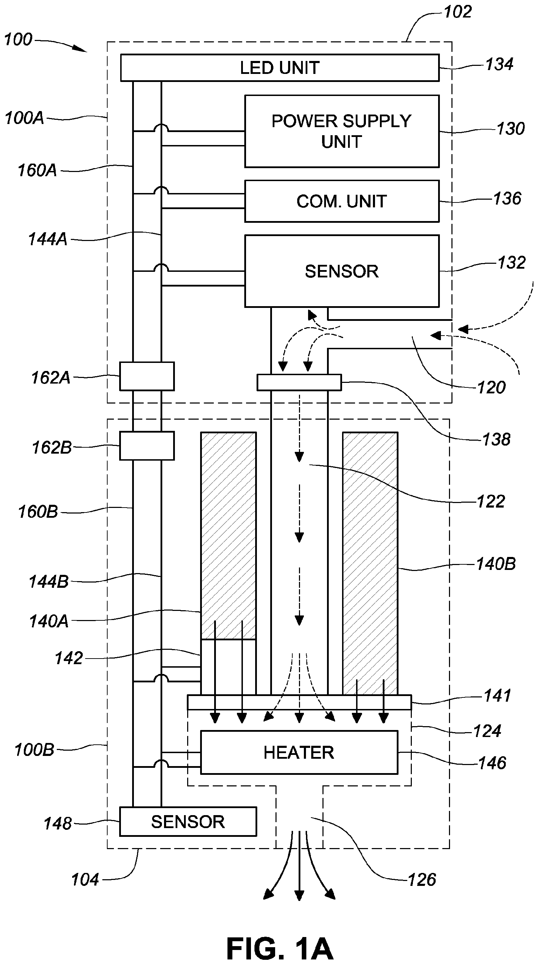

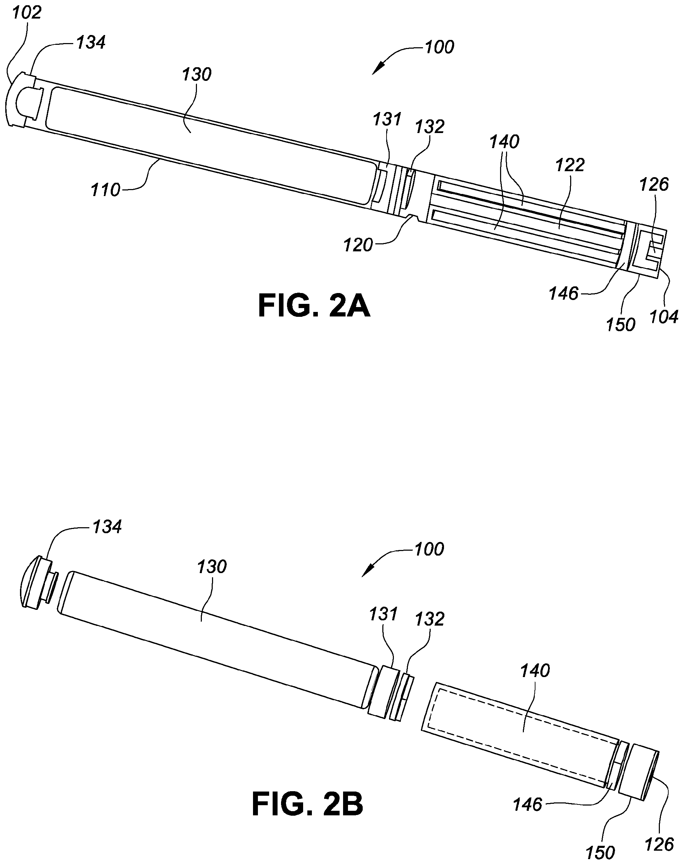

FIG. 1A shows a structural overview of an electronic smoking device (ESD) 100 constructed according to the principles of the disclosure. The ESD 100 may be disposable or reusable. The ESD 100 may have a multi-body construction including two or more bodies. For example, the ESD 100 may be a reusable ESD including a first body 100A and a second body 100B and/or the like, that may be easily connected to and disconnected from each other anytime without using any special tools. For example, each body may include threaded parts. Each body may be covered by a different housing. The second body 100B may contain consumable material, such as, *e.g*., smoking liquid and/or the like. When the consumable material is fully consumed, the second body 100B may be disconnected from the first body 100A and replaced with a new one. Also, the second body 100B may be replaced with another one with a different flavor, strength, type and/or the like. Alternatively, the ESD 100 may have a single body construction, as shown in FIG. 2A. Regardless of the construction type, the ESD 100 may have an elongated shape with a first end 102 and a second end 104, as shown in FIG. 2A, which may be similar to a conventional cigarette shape. Other non-conventional cigarette shapes are also contemplated. For example, the ESD 100 may have a smoking pipe shape or the like.

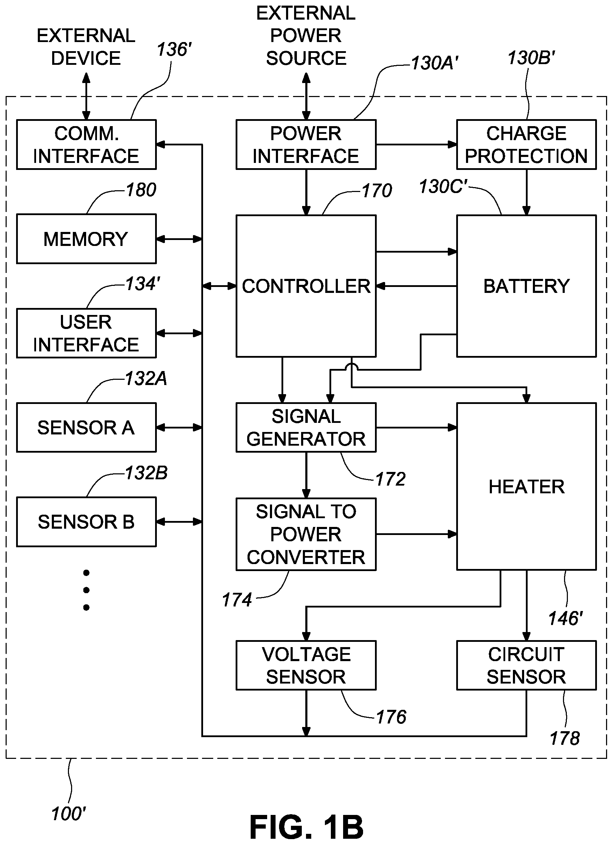

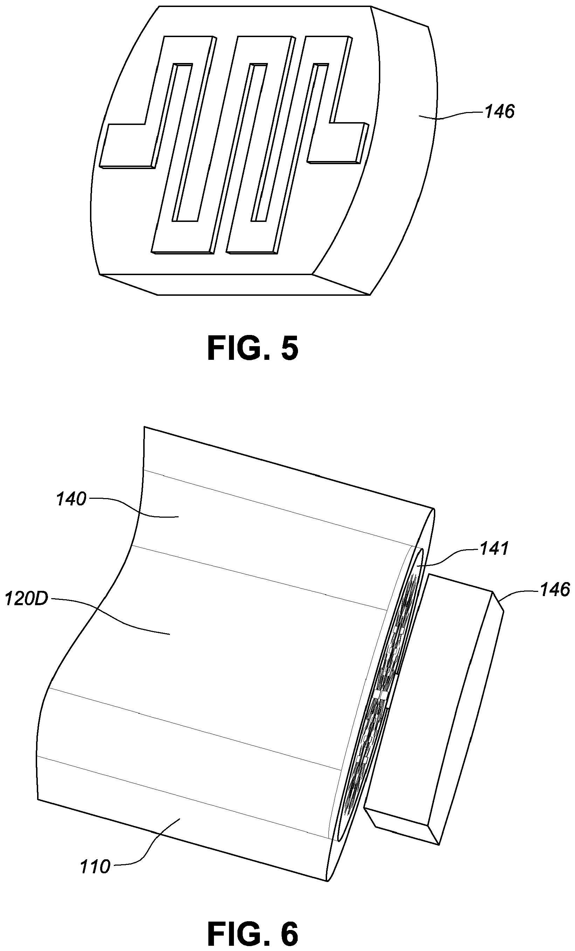

The ESD 100 may include an air inlet 120, an air flow path 122, a vaporizing chamber 124, a smoke outlet 126, a power supply unit 130, a sensor 132, a container 140, a dispensing control device 141, a heater 146, and/or the like. Further, the ESD 100 may include a controller, such as, *e.g*., microcontroller, microprocessor, a custom analog circuit, an application-specific integrated circuit (ASIC), a programmable logic device (PLD) (*e.g*., field programmable gate array (FPGA) and the like) and/or the like and basic digital and analog circuit equivalents thereof, which is explained below in detail with reference to FIG. 1B. The air inlet 120 may extend from, for example, an exterior surface of the housing 110 as shown in FIG. 2A. The air flow path 122 may be connected to the air inlet 120 and extending to the vaporizing chamber 124. The smoke outlet 126 may be connected to the vaporizing chamber 124. The smoke outlet 126 may be formed at the second end 104 of the ESD 100 and connected to the vaporizing chamber 124. When a user sucks the second end 104 of the ESD 100, air outside the air inlet 120 may be pulled in and moved to the vaporizing chamber 124 via the air flow path 122, as indicated by the dotted arrows in FIG. 1. The heater 146 may be a solid state heater shown in FIG. 5 or the like, and located in the vaporizing chamber 124. The container 140 may contain the smoking liquid and connected to the vaporizing chamber 124. The container 140 may have an opening connected to the vaporizing chamber 124. The container 140 may be a single container or a group of containers, such as, *e.g*., containers 140A, 140B and the like, that are connected to or separated from each other.

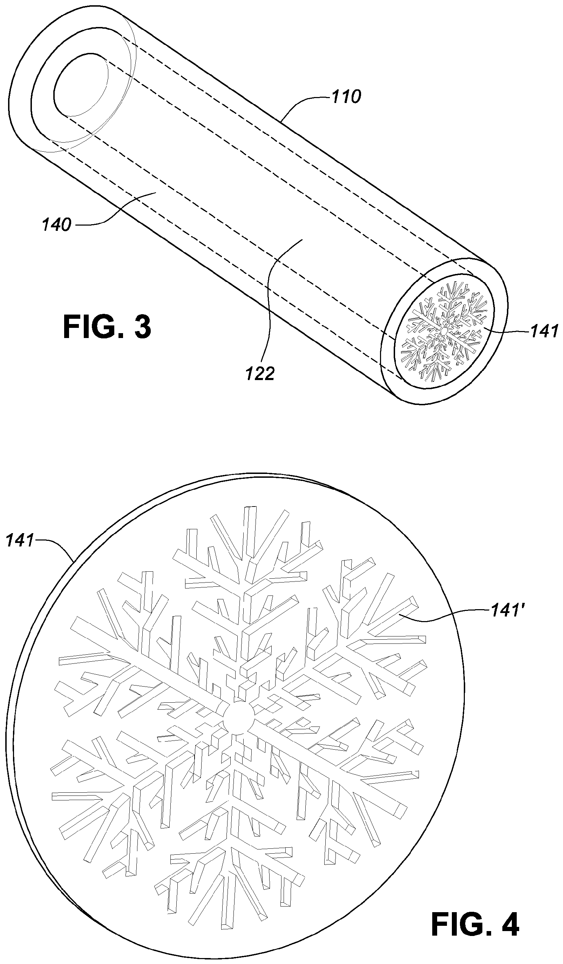

The dispensing control device 141 may be connected to the container 140 in order to control flow of the smoking liquid from the container 140 to the vaporizing chamber 124. When the user is not smoking the ESD 100, the dispensing control device 141 may not dispense the smoking liquid from the container 140, which is described below in detail with reference to FIGS. 3 and 4. The dispensing control device 141 may not need any electric power from, for example, the power supply unit 130 and/or the like, for operation.

In one aspect, the dispensing control device 141 may be a micro liquid screen 141, such as, *e.g*., micro-etched screen, micromesh screen and the like. As shown in FIG. 4, the micro liquid screen 141 may have a micro aperture pattern 141', which may keep the smoking liquid from seeping out therethrough by a surface tension and/or the like when the ESD 100 is not being used or when an air flow within the vaporizing chamber 124 is minimal. When an external force is applied, the smoking liquid may flow through the micro liquid screen 141. For example, when the user sucks the second end 104 of the ESD 100, an air flow may be formed in the vaporizing chamber 124 from the air flow path 122 to the smoke outlet 126, which may temporarily break the surface tension of the smoking liquid formed at the micro aperture pattern 141' of the at the micro liquid screen 141. When the air flow is discontinued, the surface tension may be reestablished at the micro aperture pattern 141' of the micro liquid screen 141, and the smoking liquid may stop being drawn therethrough. The micro liquid screen 141 may have a circular shape with a diameter larger than that of the container 140. One side of the micro liquid screen 141 may face an opening of the container 140 and the air flow path 122, and the other side may face the vaporizing chamber 124 and the heater 141.

The micro liquid screen 141 may be a passive device that does not require electric power and a control signal. Other passive or active filtering/screening devices are also contemplated for the dispensing control device 141. For example, the dispensing control device may be a semi-active dispensing device, such as, *e.g*., electro-permeable membrane or the like, which does not allow a liquid to flow therethrough unless an electrical field is applied thereto. Alternatively or additionally, an active dispensing device 142 may be connected to the container 140 in order to consistently dispense substantially the same amount of smoking liquid to the vaporizing chamber 124 each time. As shown in FIG. 6 the dispensing control device 141 and the heater 146 may be located adjacent to each other with a very small gap therebetween, in order to efficiently vaporize the smoking liquid.

The power supply unit 130 may be connected to one or more components that require electric power, such as, *e.g*., the sensor 132, the active dispensing device 142, the heater 146, and the like, via a power bus 160. The power supply unit 130 may include a battery (not shown), such as, *e.g*., a rechargeable battery, a disposable battery and/or the like. The power unit 130 may further include a power control logic (not shown) for carrying out charging of the battery, detecting the battery charge status, performing power save operations and/or the like. The power supply unit 130 may include a non-contact inductive recharging system such that the ESD 100 may be charged without being physically connected to an external power source. A contact charging system is also contemplated

The sensor 132 may be configured to detect the user's action for smoking, such as, *e.g*., sucking of the second end 104 of the ESD 100, touching of a specific area of the ESD 100 and/or the like. When the user's action for smoking is detected, the sensor 132 may send a signal to other components via a data bus 144. For example, the sensor 132 may send a signal to turn on the heater 146. Also, the sensor 132 may send a signal to the active dispensing device 142 (if utilized) to dispense a predetermined amount of the smoking liquid to the vaporizing chamber 124. When the smoking liquid is dispensed from the container 140 and the heater 146 is turned on, the smoking liquid may be mixed with the air from the air flow path 122 and vaporized by the heat from the heater 146 within the vaporizing chamber 124. The resultant vapor (*i.e*., smoke) may be pulled out from the vaporizing chamber 144 via the smoke outlet 126 for the user's oral inhalation, as indicated by solid arrows in FIG. 1. In order to prevent the smoke generated in the vaporizing chamber 144 from flowing towards the air inlet 120, the air flow path 122 may include a backflow prevention screen or filter 138.

When the user's action for smoking is stopped, the sensor 132 may send another signal to turn off the heater 146, the active dispensing device 142, and/or the like, and vaporization and/or dispensing of the smoking liquid may stop immediately. In an alternative embodiment, the sensor 132 may be connected only to the power supply unit 130. When the user's action for smoking is detected, the sensor 132 may send a signal to the power supply unit 130. In response to the signal, the power supply unit 130 may turn on other components, such as, *e.g*., the heater 146 and the like, to vaporize the smoking liquid.

In an embodiment, the sensor 132 may be an air flow sensor. For example, the sensor 132 may be connected to the air inlet 120, the air flow path 122, and/or the like, as shown in FIG. 1. When the user sucks the second end 104 of the ESD 100, some of the air pulled in from the air inlet 120 may be moved towards the sensor 132, which may be detected by the sensor 132. Additionally or alternatively, a capacitive sensor 148 may be used to detect the user's touching of a specific area of the housing 100. For example, the capacitive sensor 148 may be formed at the second end 104 of the ESD 100. When the ESD 100 is moved to the user's mouth and the user's lip touches the second end 104, a change in capacitance may be detected by the capacitive sensor 148, and the capacitive sensor 148 may send a signal to activate the heater 146 and the like. Other types of sensors are also contemplated for detecting the user's action for smoking, including, for example, an acoustic sensor, a pressure sensor, a touch sensor, an optical sensor, a Hall Effect sensor, an electromagnetic field sensor, and/or the like.

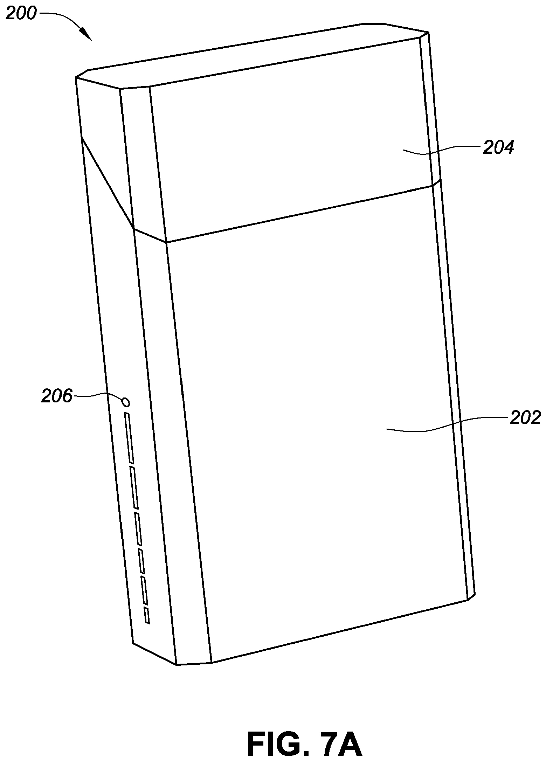

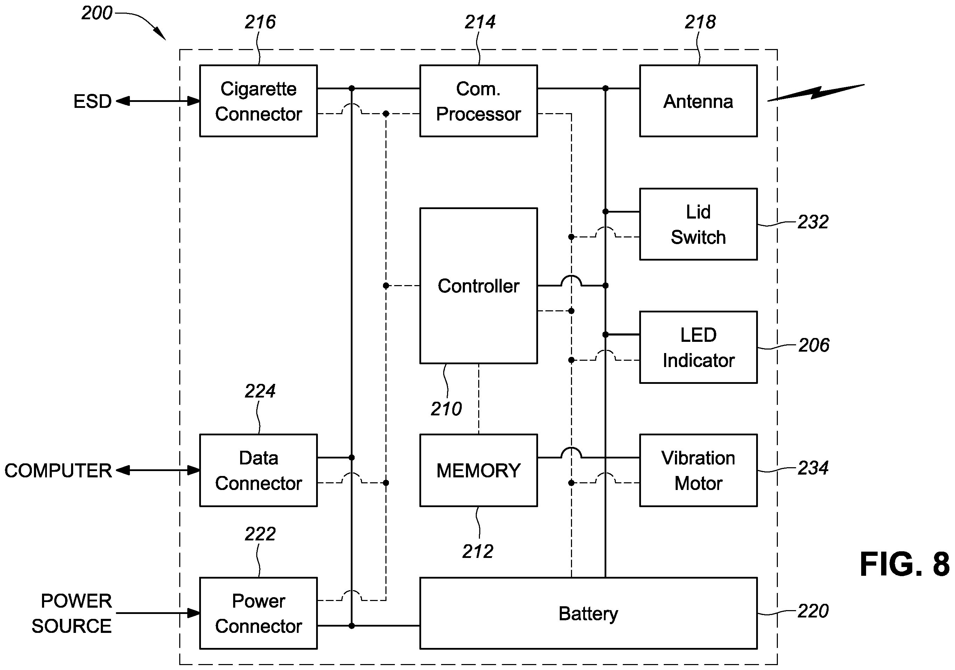

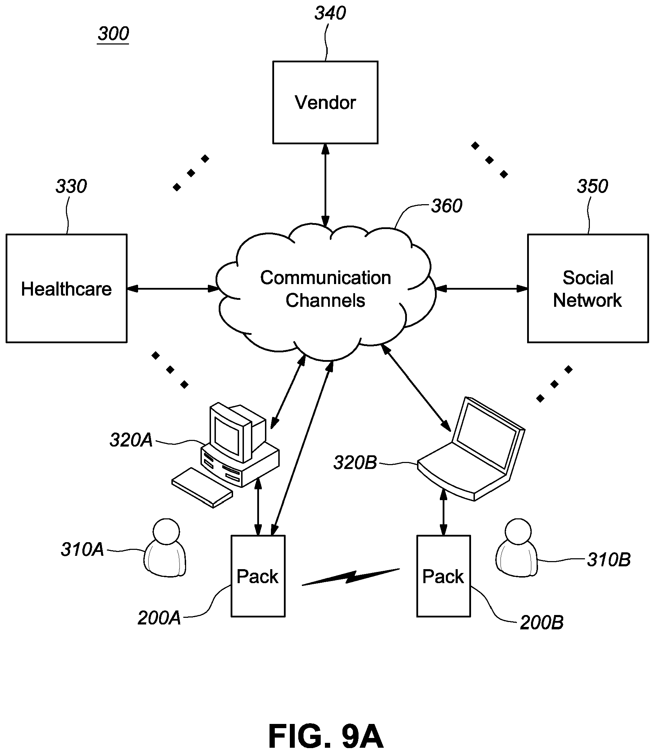

The ESD 100 may further include a communication unit 136 for wired (*e.g*., SPI (Serial Peripheral Interface) or the like) and/or wireless communications with other devices, such as, *e.g*., a pack 200 (shown in FIG. 7A) for the ESD 100, a computer 320A, 320B (shown in FIG. 9A) and/or the like. The communication unit 136 may also connect the ESD 100 to a wired network (*e.g*., LAN, WAN, Internet, Intranet and/or the like) and/or a wireless network (*e.g*., a WIFI network, a Bluetooth network, a cellular data network and/or the like). For example, the communication unit 136 may send usage data, system diagnostics data, system error data, and/or the like to the pack 200, the computer 320, and/or the like. To establish wireless communication, the communication unit 136 may include an antenna and/or the like. The ESD 100 may include a terminal 162 for wired communication. The terminal 162 may be connected to another terminal, such as, *e.g*., a cigarette connector 216 (shown in FIG. 8) of the pack 200 or the like, in order to exchange data. The terminal 162 may also be used to receive power from the pack 200 or other external power source and recharge the battery in the power supply unit 130.

When the ESD 100 has a multi-body construction, the ESD 100 may include two or more terminals 162 to establish power and/or data connection therebetween. For example, in FIG. 1, the first body 100A may include a first terminal 162A and the second body 100B may include a second terminal 162B. The first terminal 162A may be connected to a first power bus 160A and a first data bus 144A. The second terminal 162B may be connected to a second power bus 160B and a second data bus 144B. When the first and second bodies 100A and 100B are connected to each other, the first and second terminals 162A and 162B may be connected to each other. Also, the first power bus 160A and the first data bus 144A are connected to the second power bus 160B and the second data bus 144B, respectively. To charge the battery in the power supply unit 130, exchange data and/or the like, the first body 100A may be disconnected from the second body 100B and connected to the pack 200 or the like, which may, in turn, connect the first terminal 162A to the cigarette connector 216 of the pack 200 or the like. Alternatively, a separate terminal (not shown) may be provided to the ESD 100 for charging and/or wired communications with an external device.

The ESD 100 may further include one or more user interface devices, such as, *e.g*., an LED unit 134, a sound generator (not shown), a vibrating motor (not shown), and/or the like. The LED unit 134 may be connected to the power supply unit 130 via the power bus 160A and the data bus 144A, respectively. The LED unit 134 may provide a visual indication when the ESD 100 is operating. Additionally, when there is an issue and/or problem within the ESD 100, the integrated sensor/controller circuit 132 may control the LED unit 134 to generate a different visual indication. For example, when the container 140 is almost empty or the battery charge level is low, the LED unit 134 may blink in a certain pattern (*e.g*., blinking with longer intervals for thirty seconds). When the heater 146 is malfunctioning, the heater 146 may be disabled and control the LED unit 134 may blink in a different pattern (*e.g*., blinking with shorter intervals for one minute). Other user interface devices may be used to show a text, image, and/or the like, and/or generate a sound, a vibration, and/or the like.

In the ESD 100 shown in FIG. 1A, the sensor 132 alone may not be able to control the user interface devices, the communication unit 136, the sensors 132 and 148 and/or the like. Furthermore, it may not be possible to carry out more complex and sophisticated operations with the sensor 132 alone. Thus, as noted above, a controller, such as, *e.g*., microcontroller, microprocessor, a custom analog circuit, an application-specific integrated circuit (ASIC), a programmable logic device (PLD) (*e.g*., field programmable gate array (FPGA) and the like) and/or the like and basic digital and analog circuit equivalents thereof, may be included the ESD 100. For example, FIG. 1B shows a structural overview of another ESD 100' constructed according to the principles of the disclosure. The ESD 100' may include a controller 170, a signal generator 172, a signal to power converter 174, a voltage sensor 176, a current sensor 178, a memory 180, and/or the like. Further, the ESD 100' may include a power interface 130A', a charge/discharge protection circuit 130B', a battery 130C', one or more sensors (*e.g*., sensor 132A, sensor 132B and/or the like), a user interface 134', a communication interface 136', a heater 146' and/or the like, which may be similar to the components of the ESD 100 shown in FIG. 1A. Two or more components may be integrated as a single chip, a logic module, a PCB, or the like, to reduce size and manufacturing costs and simplify the manufacturing process. For example, the controller 170 and a sensor 132A may be integrated as a single semiconductor chip.

The controller 170 may perform various operations, such as, *e.g*., heater calibration, heating parameter adjustment/control, dosage control, data processing, wired/wireless communications, more comprehensive user interaction, and/or the like. The memory 180 may store instructions executed by the controller 170 to operate the ESD 100' and carry out various basic and advanced operations. Further, the memory 180 may store data collected by the controller 170, such as, *e.g*., usage data, reference data, diagnostics data, error data, and/or the like. The charge/discharge protection circuit 130B' may be provided to protect the battery 130C' from being overcharged, overly discharged, damaged by an excessive power and/or the like. Electric power received by the power interface 130A' may be provided to the battery 130C' via the charge/discharge protection circuit 130B'. Alternatively, the controller 170 may perform the charge/discharge protection operation when the charge/discharge protection circuit 130B' is not available. In this case, the electric power received by the power interface 130A' may be provided to the battery 130C' via the controller 170.

The signal generator 172 may be connected to the controller 170, the battery 130C' and/or the like, and may be configured to generate a power control signal, such as, *e.g*., a current level signal, a voltage level signal, a pulse-width modulation (PWM) signal and the like, to control the power supplied to the heater 146'. Alternatively, the power control signal may be generated by the controller 170. The converter 174 may be connected to the signal generator 172 or the controller 170 to convert the power control signal from the signal generator 172 to an electrical power provided to the heater 146. With this configuration, the power from the battery 130C' may be transferred to the heater 146' via the signal generator 172 or via the signal generator 172 and the converter 174. Alternatively, the power from the battery 130C' may be transferred to the signal generator 172 via the controller 170 and transferred to the heater 146 directly or via the signal to power converter 174.

The voltage sensor 176 and the current sensor 178 may be provided to detect an internal voltage and current of the heater 146', respectively, for heater calibration, heating parameter control and/or the like. For example, each heater 146 may have a slightly different heating temperature, which may be caused by a small deviation in resistance. To produce a more consistent unit-to-unit heating temperature, the integrated sensor/controller circuit 132 may measure a resistance of the heater 146 and adjust heating parameters (e.g., an input current level, heating duration, voltage level, and/or the like) accordingly. Also, the heating temperature of the heater 146 may change while the heater 146 is turned on. The integrated sensor 132 / controller 170 circuit may monitor a change in resistance while the heater 146 is turned on and adjust the current level in a real-time basis to maintain the heating temperature at substantially the same level. Further, the integrated sensor 132 / controller circuit 170 may monitor whether or not the heater 146 is overheating and/or malfunctioning, and disable the heater 146 for safety purposes when the heating temperature is higher than a predetermined temperature range and/or the heater 146 or other component is malfunctioning.

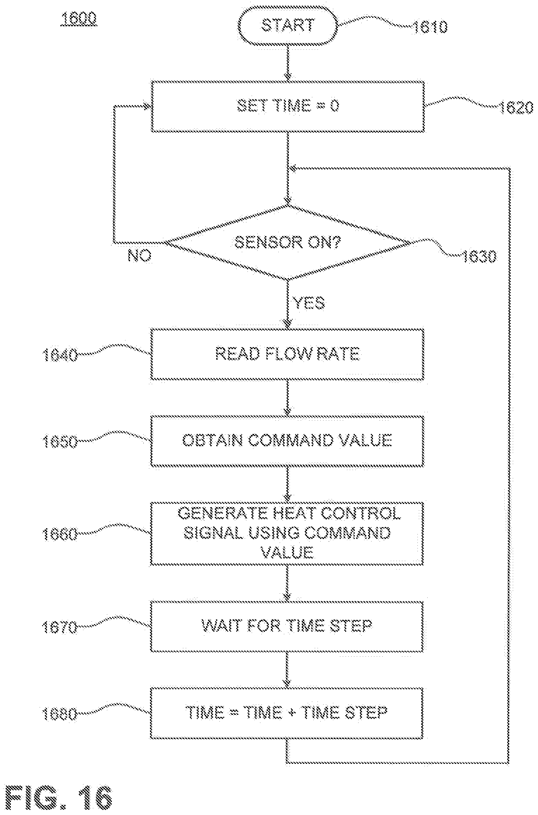

For example, FIGS. 16, 17, 18, 19, 20 and 21 show various processes for carrying out advanced functionalities in the ESD 100 or ESD 100' according to the principles of the disclosure. FIG. 16 shows a flowchart for a process 1600 for heater characterization based heat control refinement according to the principles of the disclosure. Upon starting the process 1600 (at 1610), TIME may be set to zero (0) (at 1620). When the sensor 132 (*i.e*., air flow sensor) is not on (NO at 1630), the process 1600 may move back to set TIME to zero (0) (at 1620). When the sensor is on (YES at 1630), the controller 170 may read an air flow rate (at 1640). Then the controller 170 may look up a characterization formula (*e.g*., one or more time versus temperature curves or the like) or table (*e.g*., lookup table or the like) based on at least one of the air flow rate and the time in order to obtain COMMAND VALUE, which is a value that the controller 170 determines to be for the heater 146 at any moment in time (at 1650). The COMMAND VALUE may then be applied to the heater 146, and the heater 146 generates heat based on the COMMAND VALUE (at 1660). The controller 170 may then wait for a time period TIME STEP (*e.g*., 1 second) (at 1670), which defines a time interval between the air flow rate reading (at 1640) and the TIME STEP is added to the current TIME (at 1680) and the process 1600 may move back to checking whether the sensor 132 is on or not (at 1630).

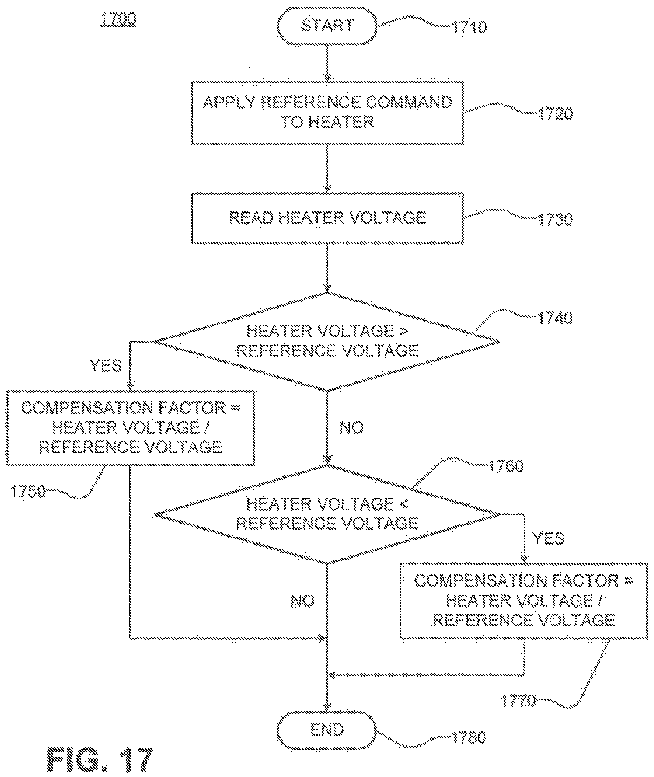

FIG. 17 shows a flowchart for process 1700 for heater self-calibration control according to the principles of the disclosure. Upon starting the process 1700 (at 1710), REFERENCE COMMAND may be applied to the heater 146. The REFERENCE COMMAND may be a heater command value established by the manufacturer to test a resistance of the heater 146. Then the voltage sensor 176 may read the internal voltage (*i.e*., HEATER VOLTAGE) of the heater 146 (at 1730). The HEATER VOLTAGE may be then compared to REFERENCE VOLTAGE, which may be a normal voltage drop expected to be measured across the heater 146 based on the characterization of the heater wire at the manufacturer. When the HEATER VOLTAGE is greater than the REFERENCE VOLTAGE (YES at 1740), the value of the HEATER VOLTAGE divided by the REFERENCE VOLTAGE may be set as COMPENSATION FACTOR (at 1750), which may be a value, by which future heater commands may be multiplied for the purpose of compensating for inconsistency of the heater resistance value among the heaters. The COMPENSATION FACTOR may be initially set to one (1). When the HEATER VOLTAGE is smaller than the REFERENCE VOLTAGE (NO at 1740, YES at 1760), the value of the HEATER VOLTAGE divided by the REFERENCE VOLTAGE may be set as the COMPENSATION FACTOR (at 1770). When the HEATER VOLTAGE is not greater than the REFERENCE VOLTAGE (NO at 1740) and not smaller than the REFERENCE VOLTAGE (NO at 1760), there may be no change in the COMPENSATION FACTOR and the process 1700 may terminate (at 1780).

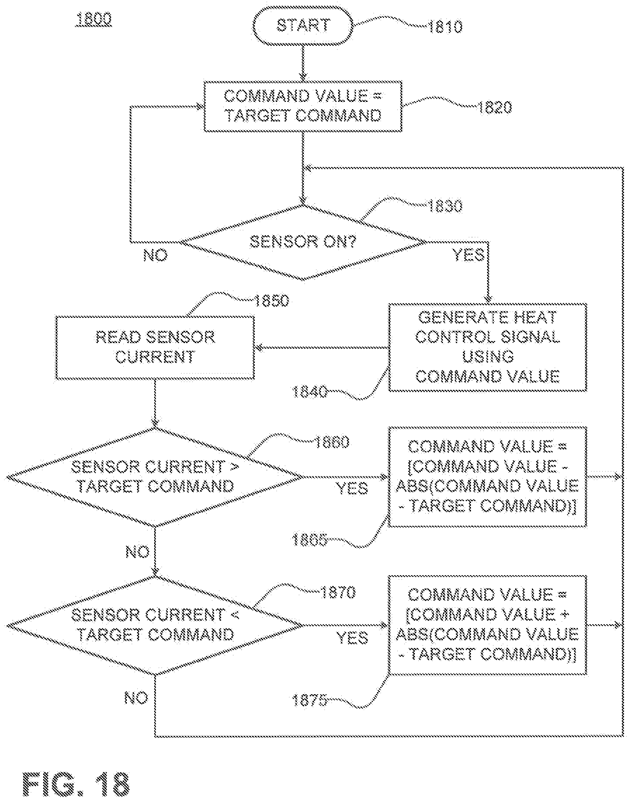

FIG. 18 shows a flowchart for a process 1800 for current monitoring based heater control according to the principles of the disclosure. Upon starting the process (at 1810), TARGET COMMAND may be set as COMMAND VALUE (at 1820). The TARGET COMMAND may be a constant that sets a target heat command for the heater 146 typically based on characterizations of the heater wire at the manufacturer. The COMMAND VALUE may be a value that the controller 170 may send to the heater 146. The COMMAND VALUE may be a value that the controller 170 believes the heater command should be at any moment in time. When the sensor 132 (*e.g*., inhalation sensor) is turned off (NO at 1830), the process 1800 may move back to step 1820. When the sensor 132 is turned on (YES at 1830), a heater control signal may be generated based on the COMMAND VALUE (at 1840) and the current sensor 178 may read an internal current of the heater 146 (at 1850) and store it as SENSOR CURRENT. Then, the SENSOR CURRENT may be compared to the TARGET COMMAND (at 1860, 1870). WHEN the SENSOR CURRENT is greater than the TARGET COMMAND (YES at 1860), an absolute value of COMMAND VALUE-(COMMAND VALUE-TARGET COMMAND) may be set as a new COMMAND VALUE (at 1865) and the process 1800 may move to step 1830. When the SENSOR CURRENT is smaller than the TARGET COMMAND (NO at 1860, YES at 1870), an absolute value of COMMAND VALUE + (COMMAND VALUE-TARGET COMMAND) may be set as the new COMMAND VALUE (at 1875) and the process 1800 may move to step 1830. When the SENSOR CURRENT is not greater than and not smaller than the TARGET COMMAND (NO at 1860, NO at 1870), no change may be made to the COMMAND VALUE and the process 1800 may move to step 1830.

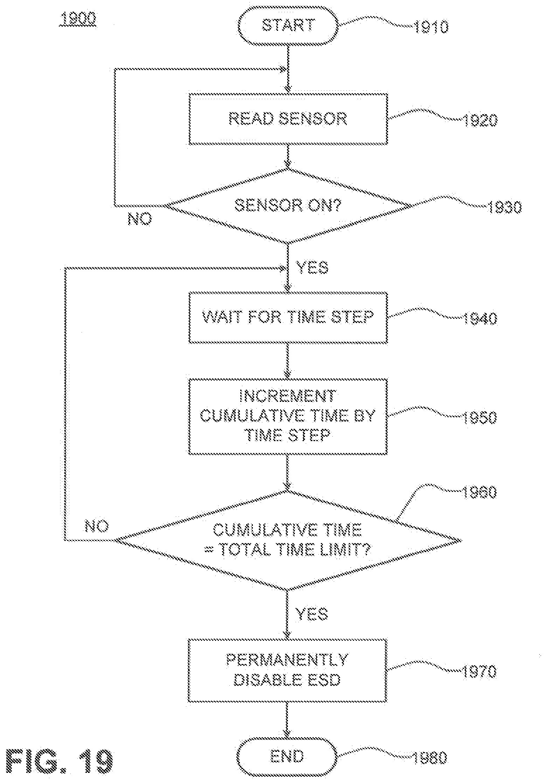

FIG. 19 shows a flowchart for a process 1900 for limiting smoking liquid deterioration and contamination after the first use according to the principles of the disclosure. Upon starting the process 1900 (at 1910), the controller may read the sensor 132 (at 1920) to check whether the sensor 132 is on or not. When the sensor 132 is not on (NO at 1930), the process 1900 may move back to read the sensor 132 (at 1920). When the sensor 132 is on (YES at 1930), the controller 170 may wait for a predetermined period of time TIME STEP (at 1940) and increment CUMULATIVE TIME by the TIME STEP (at 1950). The CUMULATIVE TIME may be a count value that indicates a total period of time since the heater 146 was first activated during the life of the ESD 100'. Then the CUMULATIVE TIME may be compared to TOTAL TIME LIMIT, which is a constant that sets an upper limit for the total period of time that may elapse between a first use and a last use of the ESD 100'. When the CUMULATIVE TIME has not reached the TOTAL TIME LIMIT (NO at 1960), and the process 1900 may move back to step 1940. When the CUMULATIVE TIME has reached the TOTAL TIME LIMIT (YES at 1960), the ESD 100' may be disabled permanently (at 1970), and the process 1900 may terminate at 1980.

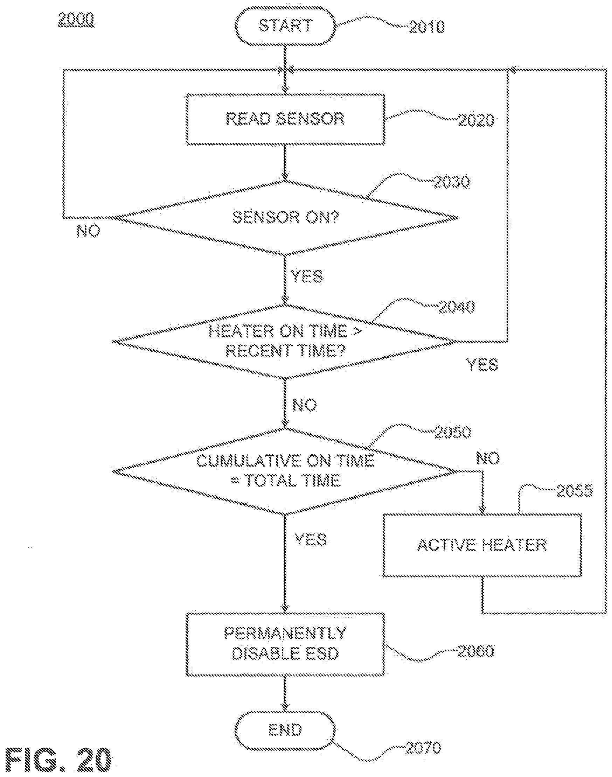

FIG. 20 shows a flowchart of a process 2000 for simplified dosage and/or heater control according to the principles of the disclosure. Upon starting the process 2000, the controller 170 may read the sensor 132 (at 2020). When the sensor 132 is not on (NO at 2030), the controller 170 may keep reading the sensor 132 (at 2020). When the sensor 132 is on (YES at 2030), the controller 170 may compare HEATER ON TIME and RECENT TIME. The HEATER ON TIME may indicate a period of time the heater 146 has been turned on since the last time the heater 146 has been turned off. The RECENT TIME may be a constant that sets a limit for the time period the heater 146 may stay turned on during any given period of time, thereby establishing a limit for the dosage per unit time that may be delivered. When the HEATER ON TIME is greater than the RECENT TIME (YES at 2040), the process 2000 may move to reading the sensor 132 (at 2020). When the HEATER ON TIME is smaller than the RECENT TIME (NO at 2040), a CUMULATIVE ON TIME may be compared to TOTAL TIME (at 2050). THE CUMULATIVE ON TIME may be a count value that indicates the total time the heater has been turned on during the product life of the ESD 100'. The TOTAL TIME may be a constant that sets a total period of time the heater 146 may stay turned on the product life of the ESD 100'. When the CUMULATIVE ON TIME has not reached the TOTAL TIME (NO at 2050), the heater 146 may be turned on (at 2055) and the process 2000 may move back to step 2020. When the CUMULATIVE ON TIME has reached the TOTAL TIME (YES at 2050), the ESD 100' may be permanently disabled (at 2060), and the process 2000 may terminate (at 2070).

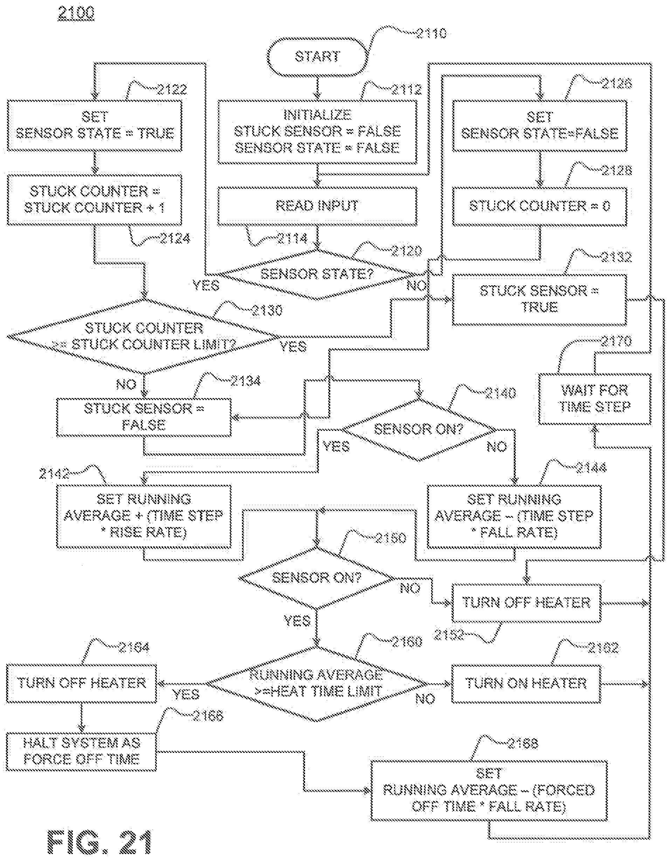

FIG. 21 shows a flowchart of a process 2100 for stuck sensor checking, heater temperature control, and forced system halting according to the principles of the disclosure. Upon starting the process 2110, the controller 170 may initialize both a stuck sensor value STUCK SENSOR and a sensor state SENSOR STATE by indicating them as false values (at 2112), and read an input of the sensor 132 (at 2114). When the SENSOR STATE is positive (YES at 2120), the controller 170 may set the SENSOR STATE as a true value, increment a stuck counter value STUCK COUNTER by the factor of one (1) (at 2124). When the STUCK COUNTER is equal to or larger than a stuck counter limit value STUCK COUNTER LIMIT (YES at 2130), the controller 170 may set the STUCK SENSOR as a true value (at 2132). When the STUCK COUNTER is not equal to or larger than the STUCK COUNTER LIMIT (NO at 2130), the controller 170 may set the STUCK SENSOR as a false value (at 2134). When the SENSOR STATE is negative (NO at 2120), the controller 170 may set the SENSOR STATE as a false value (at 2126), initialize the STUCK COUNT to zero (0) (at 2128), which may complete stuck sensor checking.

After setting the STUCK SENSOR as a true value (at 2132), the controller 170 may turn off the heater 146 (at 2152). Alternatively, after setting the STUCK SENSOR as a false value (at 2134), the controller 170 may check whether or not the sensor 132 is on (at 2140). When the sensor 132 is on (YES at 2140), a value of TIME STEP multiplied by RISE RATE may be added to a running average value RUNNING AVERAGE (at 2142). When the sensor 132 is not on (NO at 2140), the value of TIME STEP multiplied by RISE RATE may be subtracted from the RUNNING AVERAGE (at 2144). Then, the controller 170 may check whether or not the sensor 132 is on (at 2150). When the sensor 132 is not on (NO at 2150), the controller 170 may turn off the heater 146 (at 2152) and wait for the TIME STEP (at 2170), and the process 2100 may move back to read the sensor input (at 2114). When the sensor 132 is on (YES at 2150), the controller 170 may check whether the RUNNING AVERAGE is equal to or larger than a heat time limit value HEAT TIME LIMIT (at 2160). When the RUNNING AVERAGE is not equal to or larger than the HEAT TIME LIMIT (NO at 2160), the controller 170 may turn on the heater 146 and the process 2100 may move to step 2170. When the RUNNING AVERAGE is equal to or larger than the HEAT TIME LIMIT (YES at 2160), the controller 170 may turn off the heater 146 (at 2164) and forcefully halt the ESD 100' for a period FORCE OFF TIME (at 2166). Then, the value of the FALSE OFF TIME multiplied by the FALL RATE may be subtracted from the RUNNING AVERAGE (at 2168) and the process 2100 may move to step 2170. Accordingly, the controller 170 may execute this process to avoid issues of the sensor being stuck and control the temperature of the heater 146.

FIG. 2A shows a cross-section view of an exemplary design of the ESD 100 shown in FIG. 1, constructed according to the principles of the disclosure. FIG. 2B shows an exploded view of the ESD 100 shown in FIG. 2A. As noted above, the ESD 100 shown in FIGS. 2A and 2B may a single body construction and covered by a single housing 110 such that the ESD 100 may not be accidentally disassembled or broken into pieces. Further, the single body construction may be easier and less costly to design and manufacture. Thus, the single body construction may be more suitable for a disposable ESD.

Referring to FIGS. 2A and 2B concurrently, the housing 100 may have an elongated tubular shape with the LED unit 134 formed at the first end 102 and the smoke outlet 126 formed at the second end 104. The air inlet 120 may extend inwardly from the housing 110 and may be connected to the air flow path 122. The ESD 100 may further include a wall structure 131 in order to completely separate a compartment that contains the battery 130 from the air inlet 120, the air flow path 122, the container 140 and/or the like such that components in each section may be safely sealed off from each other and functions of the components may be isolated from each other. The smoke outlet 126 may be formed at a tip end piece 150. The heater 146 may be fixed by pushing the tip end piece 150 into an opening of the housing 110 at the second end 104.

In one aspect, the container 140 may surround the air flow path 122. More specifically, as shown in FIG. 3, the container 140 may have an elongated tubular shape and surrounded by the housing 110. The air flow path 122 may extend along the center of the container 140. The air flow path 122 may also have an elongated tubular shape with a smaller diameter. The housing 110, the container 140 and the air flow path 122 may be concentric. As noted above, the air flow path 122 may be connected to the air inlet 120 at one end and the other end may be connected to the vaporizing chamber 124. The container 140 may also be connected to the vaporizing chamber 124. In order to control dispensing of the smoking liquid from the container 140 to the vaporizing chamber 124, the dispensing control device 141 may be formed between the container 140 and the vaporizing chamber 124.

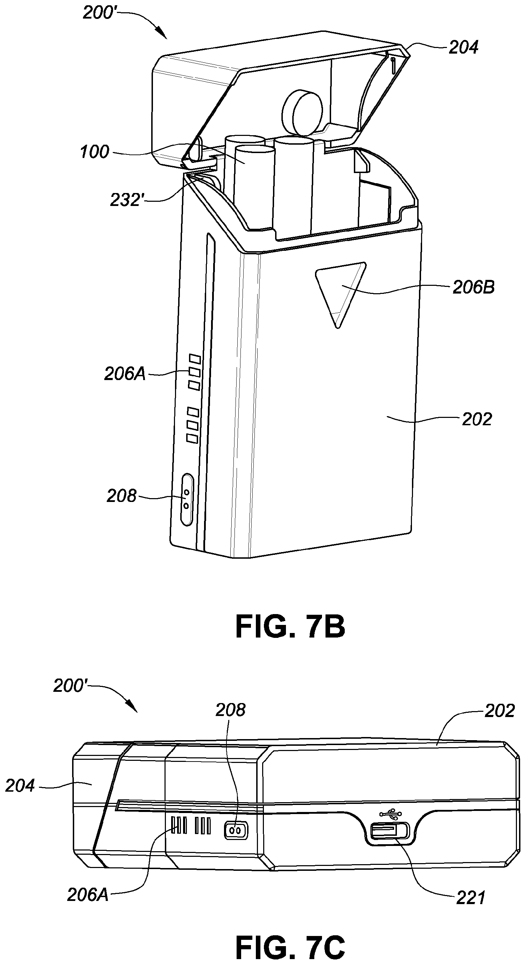

FIG. 7A shows a perspective view of the pack 200 for an ESD, constructed according to the principles of the disclosure. The pack 200 may have a conventional cigarette pack shape but other shapes are also contemplated. FIG. 7B shows a perspective view of another pack 200' and FIG. 7C shows a bottom perspective view of the pack 200' shown in FIG. 7B. FIG. 8 shows a structural overview of the pack 200 of FIG. 7A and the pack 200' of FIGS. 7B and 7C, constructed according to the principles of the disclosure. Referring to FIGS. 7A, 7B, 7C and 8 concurrently, the pack 200 may include a main body 202, a lid 204, one or more user interface devices (e.g., an indication light 206 (in FIG. 7A), 206A and 206B (in FIG. 7B), a switch 208, a vibration motor 234 (shown in Fig 8), a further display (not shown), a sound device (not shown) and/or the like), one or more connectors (e.g., a cigarette connector 216, a power connector 222, a data connector 224 and/or the like) and/or the like. The pack 200 may further include a controller 210, a memory 212, a communication processor 214, an antenna 218, a battery 220, a lid switch 232, a lid switch plunger 232', and/or the like. The lid switch plunger 232' may be connected to the lid switch 232 and configured to detecting opening and closing of the lid 204.

As noted above, the cigarette connector 216 may be connected to the terminal 162 of the ESD 100 to charge the battery in the power supply unit 130, exchange data with the integrated sensor/controller circuit 132 and/or the like. The terminal 162 and the cigarette connector 216 may be connected by a threaded type connection. Other connection types are also contemplated, including, such as, *e.g*., a non-threaded type connection, a stationary connection, a push-in (pressing) connection, and/or the like. The power connector 222 may be connected to an external power source (USB, transformer, or the like) to charge the battery 220. Additionally or alternatively, the pack 200 may include a non-contact inductive recharging system such that the pack 200 may be charged without being physically connected to any external power source. The battery 220 and the battery 130 in ESD 100 may be charged at different voltages. Thus, the pack 200 may include multiple internal voltage nets (not shown). The data connector 224 may be connected to, for example, the user's computer 320A, 320B (shown in FIG. 9A) and/or the like to exchange data between the pack 200 and the computer 320A, 320B. The power connector 222 and the data connector 224 may be combined. For example, the pack 200' may include a USB connector 221 (shown in FIG. 7C), a FireWire connector and/or the like, that may function as both the power connector 222 and the data connector 224.

The controller 210 may be configured to control overall operations of the pack 200 including one or more components noted above. For example, the controller 210 may carry out a power saving scheme by, for example, entering a power save mode or the like, when the power connector 222 is disconnected from an external power source and the lid 204 has not been opened for a predetermined period of time. Opening and closing the lid 204 may be detected by the lid switch 232. Also, the controller 210 may detect the battery charge level of the battery 220 in the pack 200 and the battery in the ESD 100 when the ESD 100 is connected to the pack 200. Further, the controller 210 may operate the user interface devices to indicate a status of the ESD 100 and the pack 200. For example, the controller 210 may operate the LED indicator 206 to blink with longer intervals when the ESD 100 is connected to the cigarette connector 216 and being charged by the battery 220 or an external power source. When there is a problem with the ESD 100 or the pack 200, the controller 210 may show an alert message or an error message on the display (not shown), generate an alert sound and/or the like. For example, when the container 140 is empty or the battery charge level is low in the ESD 100, the controller 210 may show a message on the display, activate the vibration motor 234 and/or the like. Further, when the heater 146 is overheating or malfunctioning, the controller 210 may control the LED indicator 206 to blink with shorter intervals, display a heater error message on the display, generate an alert sound and/or the like. In other words, any error detected in the ESD 100 may be transmitted to the pack 200. Further, when the pack 200 is connected to an external device, such as, *e.g*., computer or the like, an error message may be displayed on the external device.

The communication processor 214 may carry out wired communications via the data connector 224 and/or wireless communications via the antenna 218, which is described below in detail with reference to FIG. 9A. The memory 212 may include instructions to be executed by the controller 210 to carry out various operations. The memory 212 may further include usage information (*e.g*., smoking liquid level in the container 140, how many containers 140 have been consumed, amount of nicotine consumed, and/or the like), product information (*e.g*., model number, serial number and/or the like), user information (*e.g*., the user's name, sex, age, address, job, educational background, job, professional background, interests, hobbies, likes and don't-likes and/or the like) and/or the like. The user information may be received from the user's computer 320A, 320B via the data connector 224 or wirelessly via the antenna 218 and stored in the memory 212. Alternatively, the user information may be received by a social network website, such as, *e.g*., Facebook™, Linkedln™, Eharmony™ and/or the like, via the data connector 224 or wirelessly via the antenna 218.

The data stored in the pack 200 (*e.g*., the usage information, the product information, the user information and/or the like) may be shared with other devices and/or entities (*e.g*., vendors, healthcare service providers, social networks and/or the like). For example, FIG. 9A shows a conceptual overview of a system 300 for exchanging data of the pack 200 over various communication channels, constructed according to the principles of the disclosure. The system 300 may be a network of a plurality of communication devices, such as, *e.g.,* one or more packs 200 (*e.g*., a first pack 200A owned by a first user 310A, a second pack 200B owned by a second user 310B and/or the like), one or more computers 320 (*e.g*., a desktop PC 320A, a laptop PC 320B, a mobile phone (not shown), a personal data assistant (PDA) (not shown), a tablet PC (not shown) and/or the like) and/or the like, that are connected to each other via various wired and/or wireless communication channels 360 (*e.g*., LAN, WAN, Internet, intranet, Wi-Fi network, Bluetooth network, cellular network and/or the like). The user 310 may download and install a software application in her or his computers 320 such that the computer 320 and the pack 200 may exchange data with each other. Further, an app may be installed in the user's smartphone, which may be then connected to the pack 200 directly or via the communication channels 360.

As shown in FIG. 9A, the packs 200A and 200B may communicate directly with each other via the communication processor 214 and the antenna 218. For example, the packs 200A and 200B may exchange the user information with each other. When the users 310A and 310B have the same hobby or graduated from the same school, the controller 210 in each of the packs 200A and 200B may operate the user interface devices to notify the match. For example, the pack 200A may make a sound and/or show a message that the user 310B who graduated from the same school is near by the user 310A on the display. Additionally, a smartphone or a tablet computer running the app noted above may be used to interact with other users. Accordingly, the packs 200A and 200B may be used for social networking devices.

Further, the packs 200A and 200B may be connected directly to the wired and/or wireless communication channels 360 or indirectly via the computers 320A and 320B, respectively. Based on the usage data, the pack 200A may automatically send an order request to the vendor 340 when the user 310A needs more disposable ESDs or a new supply of the second bodies 100B. Further, the packs 200B may send usage data to the healthcare service provider 330, such as, *e.g*., a physician's office, a hospital and/or the like, such that a physician, a nurse, a hospital staff and/or the like may track and analyze nicotine consumption by the user 310B. The user 310B may also use the usage data to monitor how much she or he smokes and check whether she or he has been smoking less or more for a period time.

Furthermore, the ESD 100 may be susceptible to bacterial growth after a certain period of time. The usage data may be used to determine how long the ESD 100 has been used and automatically disable the ESD 100 when the ESD 100 has been used for a certain period of time and/or severity of the usage.

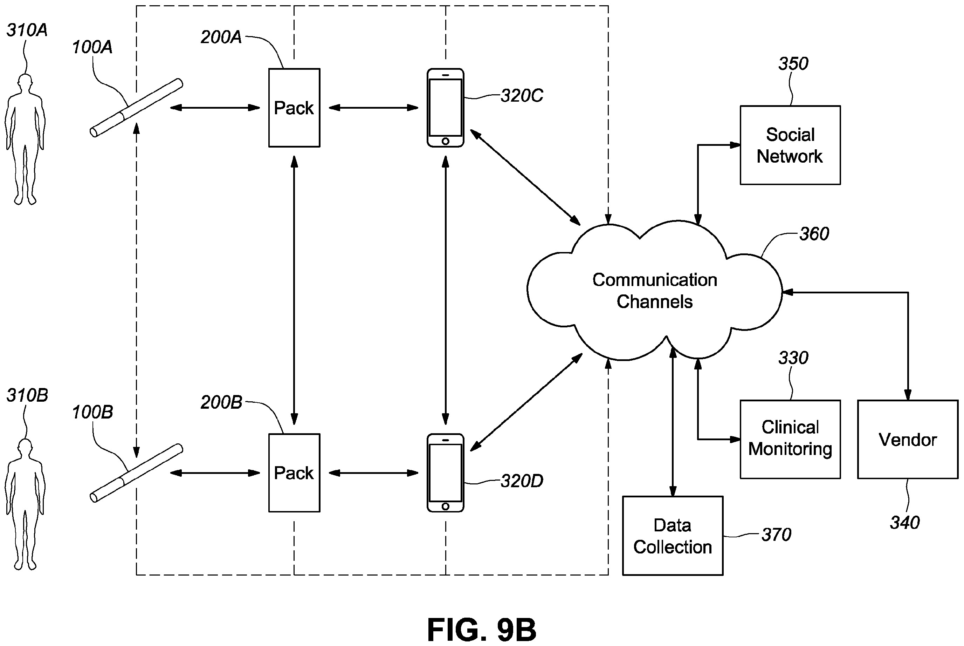

According to one embodiment, ESD 100 can be used for social networking via a "match-making" process. For example, FIG. 9B shows several possible ways in which user 310A, using ESD 100A, can connect with user 310 B, using ESD 100B. ESDs 100A and 100B can communicate with their respective packs, 200A and 200B, via a physical connection, such as when ESD 100A is placed in pack 200A or ESD 100B is placed in pack 200B. For example, data collected by ESD 100A (or 100B) (e.g., number of puffs taken, duration of the puffs, flavors used and/or the like) can be input to pack 200A (or 200B). Packs 200A and 200B can then communicate directly (e.g., by lighting up, vibrating, or making noise) or via their associated smart phones. For example, pack 200A can communicate with smart phone 320C (belonging to user 310A) via a wireless Bluetooth network or other wireless communication channels. Similarly, pack 200B can communicate with smart phone 320D (belonging to user 310B) via a wireless Bluetooth network or other wireless communication channels. Smart phones 320C and 320D can then communicate directly or via communication channels 360 (e.g., LAN, WAN, Internet, intranet, Wi-Fi network, Bluetooth network, cellular network and/or the like) that are further linked to a social network 350 (e.g., Facebook™, Linkedln™, Eharmony™ and/or the like). In an example, smart phone 320C and 320D can send each other the Facebook profiles and pictures of their respective users, 310A and 310B.

In the above "match making" embodiment of social networking, it should be noted that ESDs 100A and 100B can also communicate directly with each other, with their respective packs, with their respective phones, or with communication channels 360 (as shown by dotted lines in FIG. 9B). Matching can be performed by the ESDs, the packs, the phones, or a server or other communication channel. Currently, however, the most feasible route of communication and "match making" occurs when data from an ESD is communicated to its associated pack, and/or data from a pack is communicated to its associated phone.

In another social networking embodiment of the present disclosure, ESDs, packs, phones, and/or communication channels 360 can allow users to participate in competitions with other users. For example, users 310A and 310B can compete against each other to determine who can take the most or least puffs in one day, who can puff in the most or least places in one day, or who can make the most social connections using their ESD 100A or 100B, for example. ESDs 100A and 100B, packs 200A and 200B, and/or phones 320C and 320D, can be configured to obtain and store GPS data or other location-based services data (e.g., cellular triangulation or IP address) to allow such competitions to occur.

ESD 100 can also be used in conjunction with an application (app) for a smart phone or mobile device. For example, an app on a user's smart phone can allow the phone to receive data recorded and stored on the user's ESD 100 or pack 200. ESD 100 may record the number of puffs taken, the length of each puff, the flavors of smoke juice used, and/or the like. This data can then be input into the user's pack when ESD 100 is placed in pack 200. The smart phone app can receive such data, either directly from ESD 100 or from pack 200. Referring to FIG. 9B, the smart phone app can use communication channels 360 to share information with various vendors 340, healthcare or clinical monitoring services 330, and/or data collection services 370.

In an embodiment, a smart phone app can be used to share data with vendors 340, such as manufacturers or retailers that sell ESD 100 or smoke juice and/or other supplies and accessories used with ESD 100. For example, if a user is running low on smoke juice, this information can be communicated from the user's ESD 100 to the user's smart phone app, and then to a retailer's database. The ESD 100 or pack 200 can also contain location-based services, and the smart phone app can be configured to further relay a user's location to the retailer. The retailer may then send a coupon, promotion, or other advertisement to the user, encouraging the user to buy more smoke juice at a nearby store.

In another embodiment, a smart phone app can be used to collect data about a user characteristics (e.g., when and where they smoke, who they smoke with, how often they smoke, what times of day they smoke, triggers or deterrents for smoking, and/or the like). This user characteristic data may be very valuable to the manufacturers of ESDs 100 for research and development, marketing, and strategizing to drive sales. User characteristic data may also be useful to health care providers for purposes of clinical monitoring 330. For example, a doctor can collect data about how much or how often a patient is smoking as part of a cessation plan. Health care providers may also be interested in monitoring the amount of nicotine used in ESD 100, for example. Such information can be useful in helping patients who want to quit or cut back on smoking, or those who simply want to keep track of their smoking habits. The smart phone app can be configured to provide feedback to the user based on the user's smoking characteristics.

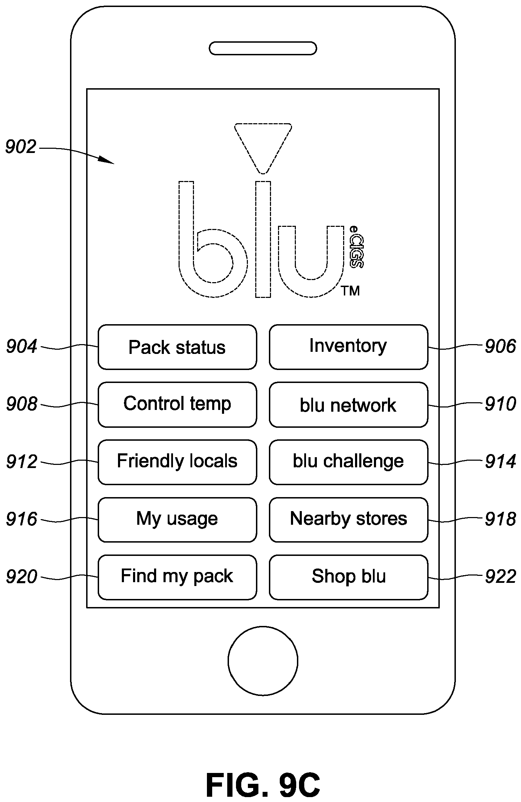

Examples of a smart phone app for use in conjunction with ESD 100 are shown in FIGS. 9C-9M. FIG. 9C shows an exemplary home screen 902 of the app, which allows a user to use the phone's touch screen to select one of the following soft buttons (a.k.a. clickable icons): pack status 904, inventory 906, control temp 908, blu network (i.e. social network for users of blu™ (hereinafter "blu") brand e-cigarettes) 910, friendly locales 912, blu challenge 914, my usage 916, nearby stores 918, find my pack 920, and shop blu 922.

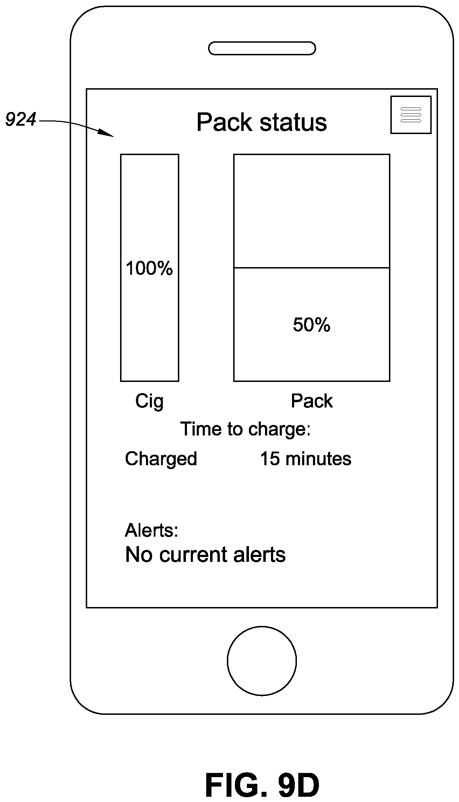

FIG. 9D shows an exemplary pack status screen 924, which can be programmed to appear when a user clicks on the pack status soft button 904. The pack status screen 924 can indicate the charge state (e.g. 0% to 100% charged) for the ESD 100 and pack 200. The time required to reach a fully charged state can also be displayed. Thus, the pack status screen 924 can function as a reminder to a user to charge his/her pack 200. In an embodiment, an alert can be displayed (and/or sounded) when the ESD 100 or pack 200 are fully charged. In another embodiment, alerts can be error codes that are displayed when errors conditions occur, such as a bad cigarette battery, a cigarette battery that is too low on charge, or a pack fault alert. The displayed error codes can be used to assist customer service in addressing the error condition.

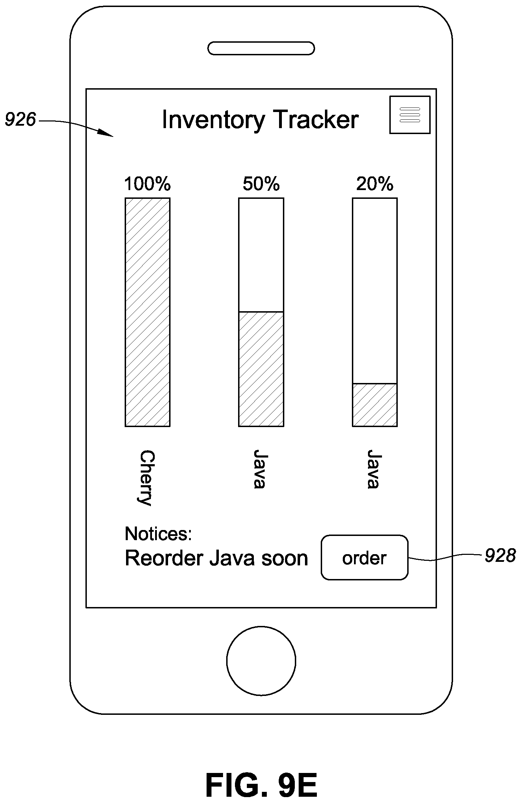

FIG. 9E shows an exemplary inventory tracker screen 926, which can be programmed to appear when a user clicks on the inventory soft button 906. The inventory tracker screen 926 can indicate the remaining levels of a user's cartomizer (e.g. second body 100B, as shown in FIG. 1A) from 0% to 100%. In addition, the inventory tracker screen can indicate if there is no cartomizer in the pack 200 or if an expired cartomizer has been loaded in the pack 200. Various notices can be displayed on the inventory tracker screen 926, such as reminders to reorder a particular type of cartomizer. Moreover, an order soft button 928 can be included on the inventory tracker screen 926.

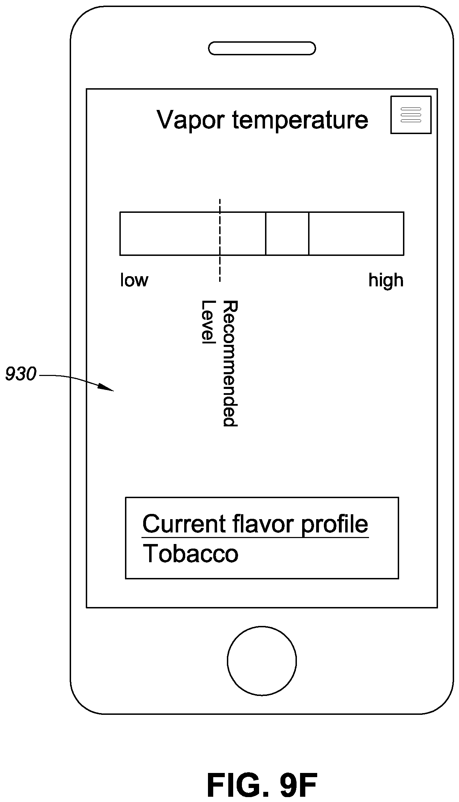

FIG. 9F shows an exemplary temperature control screen 930, which can be programmed to appear when a user clicks on the control temp soft button 908. The temperature control screen 930 can indicate the actual vapor temperature and the recommended vapor temperature for ESD 100. In addition, the temperature control screen 930 can indicate the current flavor profile for ESD 100, as different flavor profiles will have different recommended vapor temperatures.

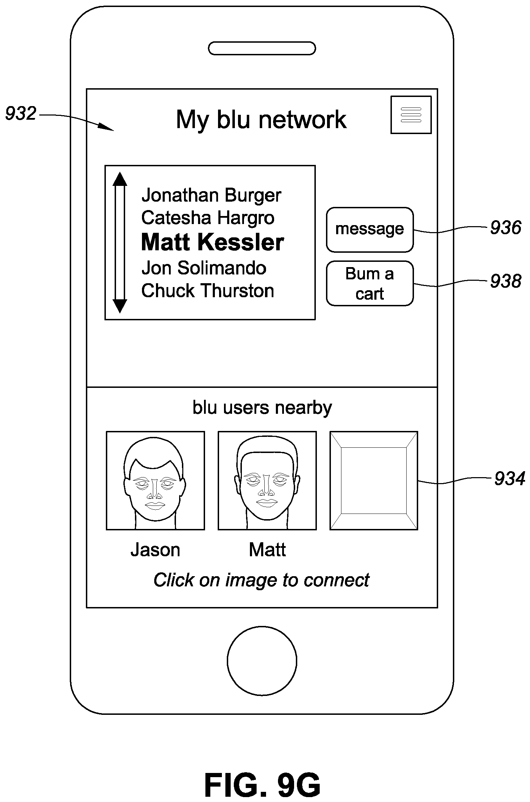

FIG. 9G shows an exemplary blu network screen 932, which is an example of a social networking screen included in the smart phone app. The blu network screen 930 can be programmed to appear when a user clicks on the blu network soft button 910. The blu network screen 932 can display a list of a user's friends, as well as images of the user's friends. An image of the user's friend can be a clickable soft button 934, which the user may select to connect with their friend (e.g. view the friend's profile). In addition, the user may click on soft button 936 to send a text or e-mail message to a friend, or soft button 938 to send a "bum a cart" request to a friend (i.e. asking the friend for a cartridge).

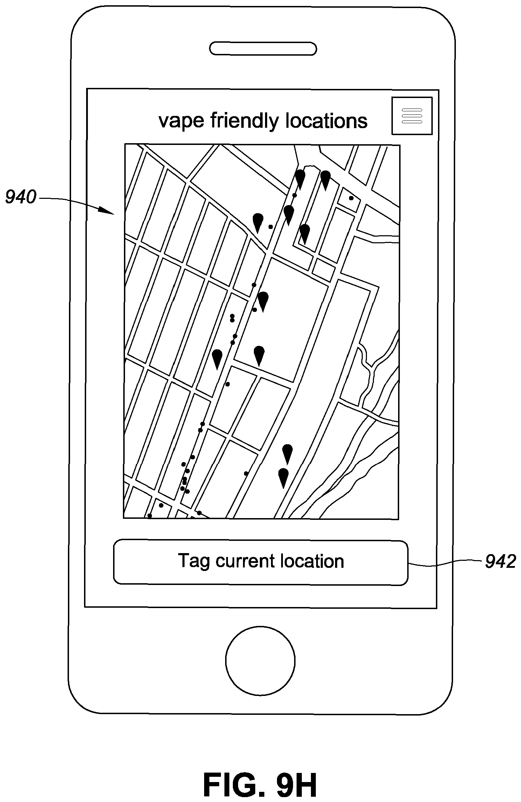

FIG. 9H shows an exemplary friendly locales screen 940, which can be programmed to appear when a user clicks on friendly locales soft button 912. The friendly locales screen 940 can be an interactive map of nearby locations where users are able to "vape," or use ESDs. In an embodiment, a user can click on soft button 942 to tag their current location and get directions to one or more vape-friendly location. In another embodiment, clicking on soft button 942 triggers a dialog to pop up, allowing a user to tag and comment on their current location.

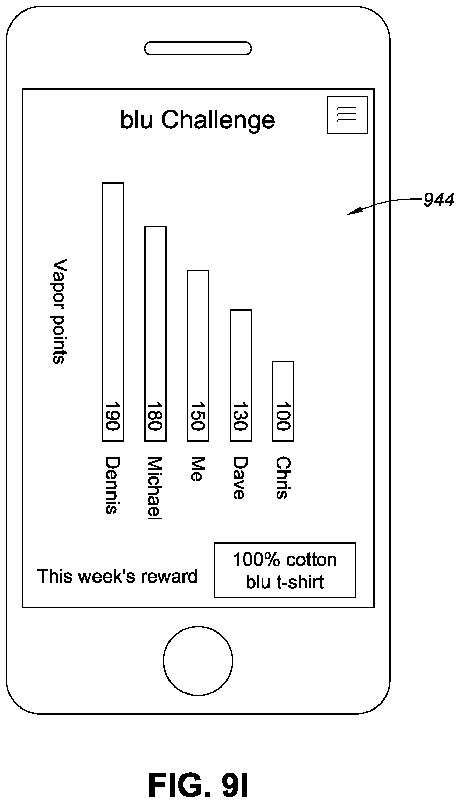

FIG. 9I shows an exemplary blu challenge screen 944, which can be programmed to appear when a user clicks on blu challenge soft button 914. The blu challenge screen is an example of a social networking application in which users can compete with other users, as discussed above with respect to FIG. 9B. For example, blu challenge screen 944 can display a user's "vapor points" in comparison to his/her friends' points. Vapor points can be earned, for example, by using a blu ESD to replace traditional cigarettes. A reward, such as a t-shirt, for example, can be given to the user with the most points. This challenge aspect of the smart phone app can also incentivize blu users to encourage users of other ESD brands to switch to blu.

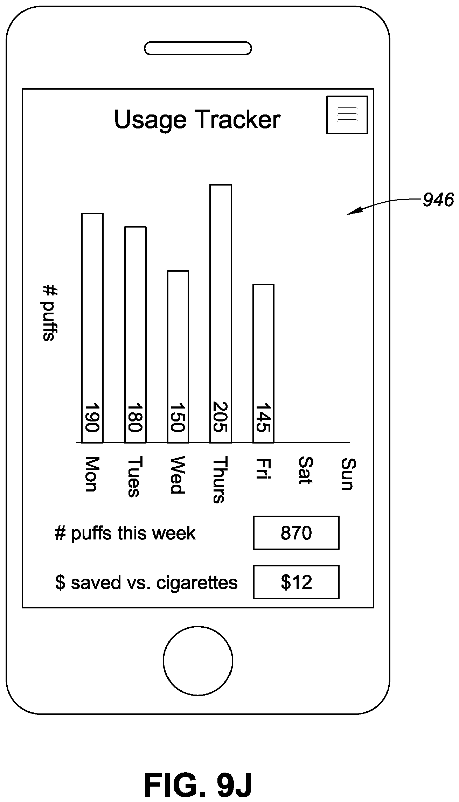

FIG. 9J shows an exemplary usage tracker screen 946, which can be programmed to appear when a user clicks on my usage soft button 916. The usage tracker screen 946 can display the number of puffs a user takes per day, per week, per month, or per year, for example. The usage tracker screen 946 can also display the cost saving of using an ESD in place of traditional cigarettes. The cost saving can be based on the number of puffs taken per day, per week, per month, or per year, for example. In an embodiment, the usage tracker screen 946 can also be used to track the number of traditional cigarettes avoided. In another embodiment, the usage tracker screen 946 can be used to track the number of puffs taken using a particular flavor.

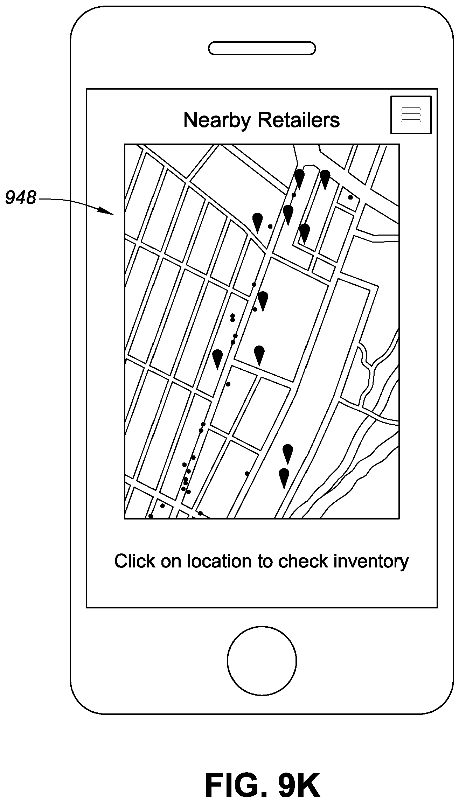

FIG. 9K shows an exemplary nearby retailers screen 948, which can be programmed to appear when a user clicks on nearby retailers soft button 918. The nearby retailers screen 948 can be an interactive map that displays retail locations in the user's vicinity. In an embodiment, the user can click on a particular retail location to check its inventory. In another embodiment, a user's phone can push a notification to the user's pack when the app detects a nearby retailer (e.g., by using GPS or other location-based services data). The pack can light up or vibrate, for example, in response to the notification. In another embodiment, a user's pack can be programmed to detect a nearby kiosk beacon and then send a notification, including the location and inventory of the kiosk, to the smart phone app. Such a notification can be displayed on nearby retailers screen 948. Notification settings (e.g., ring tone, vibration, location-based services, privacy settings, and/or the like) associated with the presently described app can be configured using the general settings application typically found in mobile devices.

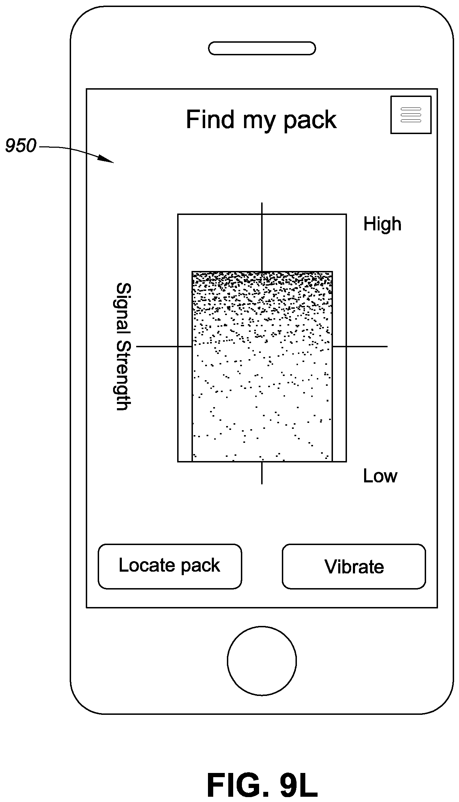

FIG. 9L shows an exemplary find my pack screen 950, which can be programmed to appear when a user clicks on the find my pack soft button 920. The app can detect the location of a user's pack based on signal strength, and the find my pack screen 950 can display the pack location using a warmer/colder metaphor. The smart phone can be configured to ring or vibrate when a user is within a given range of his/her pack.

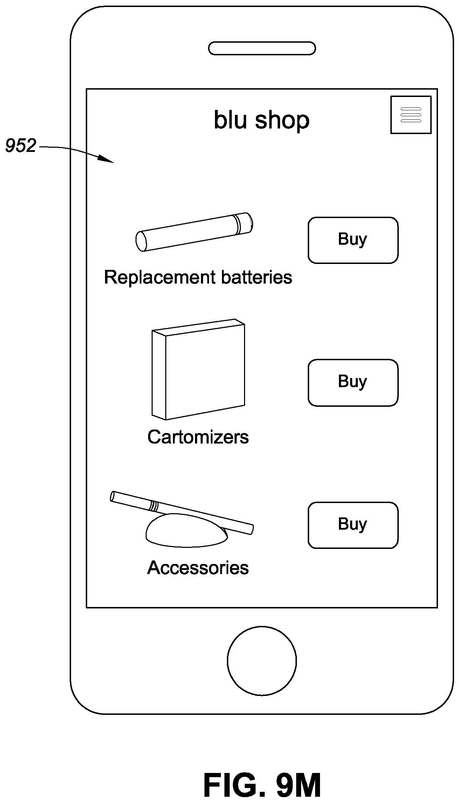

FIG. 9M shows an exemplary shop blu screen 952, which can be programmed to appear when a user clicks on the shop blu soft button 922. The shop blu screen 952 can be linked to section of an online store, such as blucigs.com, for example. Users can purchase replacement batteries, cartomizers, packs, accessories, and other ESD-related products via the shop blu screen 952.

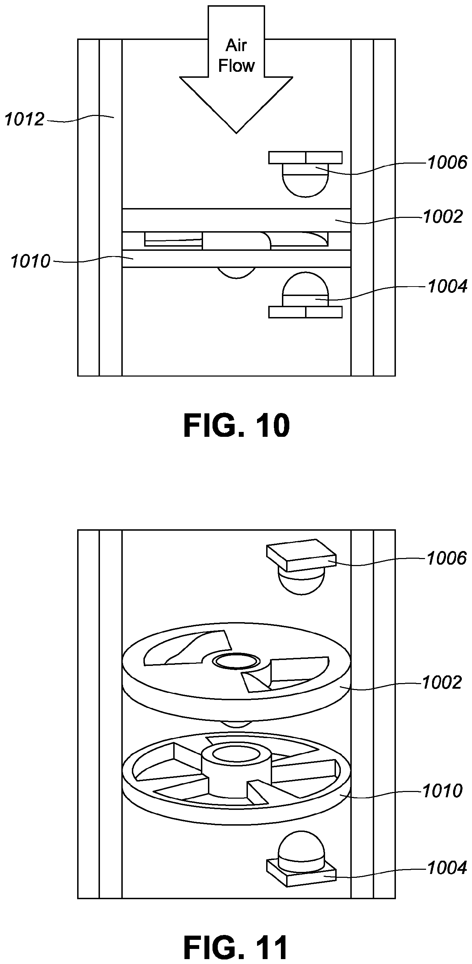

FIGS. 10 and 11 show a schematic of a sensor for the electronic smoking device constructed according to the principles of the disclosure. As shown in Figs. 10 and 11, the sensor may include an emitter (IR or visible) 1006, detector 1004, rotating disk 1002 with windows, stator 1010, and a holder 1012. The disk 1002 may have slanted windows to convert airflow into rotary thrust. Airflow causes the disk 1002 to spin. The rate of spin corresponds to airflow. The rate of spin may be detected by frequency of light pulses from the emitter device 1006 received by detector 1004. Other embodiments exist where a disk has a reflective surface. The emitter 1006 and detector 1004 are arranged on the same side of the disk 1002. The detector 1004 looks for pulses in reflections from the disk surface as the disk 1002 spins. Other embodiments exists where axis of disk is rotated 90° relative to the air flow, similar to a water wheel.

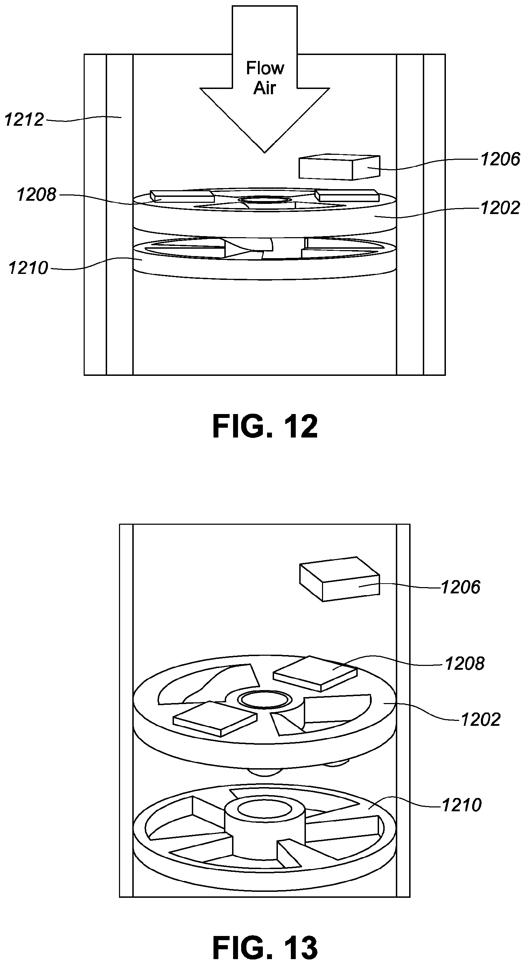

FIGS. 12 and 13 show a schematic of another sensor for the electronic smoking device constructed according to the principles of the disclosure. As shown in Figs. 12 and 13, a housing 1212 includes a stator 1210 and a disk 1202 that may have slanted windows to convert airflow into rotary thrust. Airflow causes disk 1202 to spin. The rate of spin corresponds to airflow. The rate of spin may be detected by frequency of pulses received by Hall Effect sensor 1206 from magnets 1208. Other embodiments exist where capacitive sensing regions or physical contacts are used instead of Hall Effect sensor 1206 and magnets 1208. Other embodiments exist where electric field can be applied to electromagnet (not shown). The electromagnet will prevent the disk from spinning. This provides the ability to stop airflow if so desired in order to control dosage.

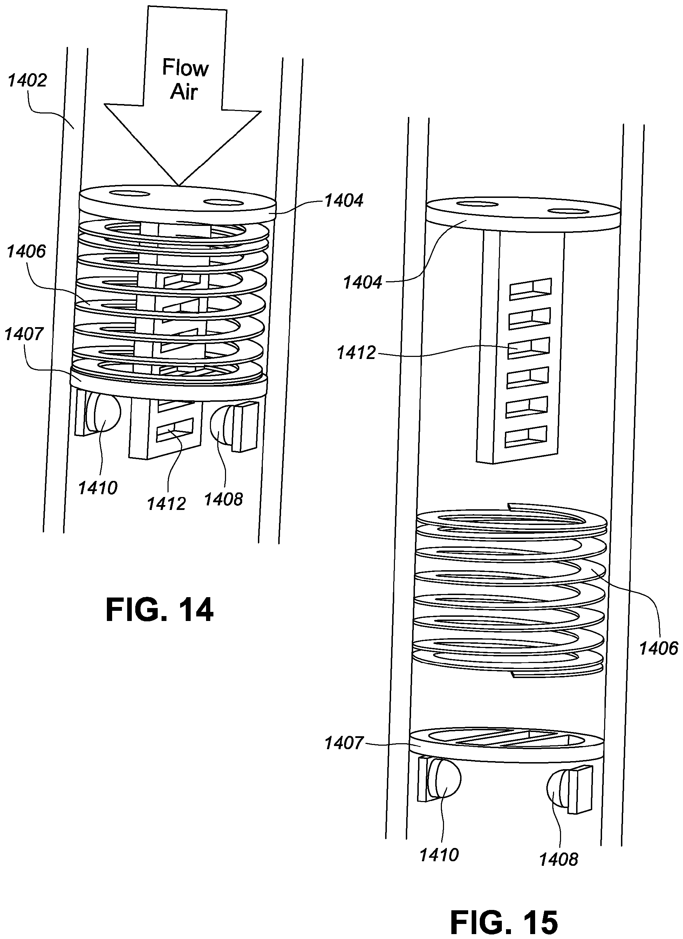

FIGS. 14 and 15 show a schematic of yet another sensor for the electronic smoking device constructed according to the principles of the disclosure. As shown in Figs. 14 and 15, the sensor includes a housing 1402, plunger with holes 1404 to allow airflow, holder 1407, spring 1406, an emitter (IR or visible) 1408, a detector 1410, and Windows 1412 in the plunger 1404. Airflow causes plunger 1404 to compress the spring 1406. The windows 1412 in the plunger 1404 produce "light/no light" condition that can be read by detector 1410. The number of windows corresponds to amount of airflow. This configuration may be used for optical sensors, capacitive sensors, hall-effect sensors and the like.

While the disclosure has been described in terms of exemplary embodiments, those skilled in the art will recognize that the disclosure can be practiced with modifications in the scope of the appended claims. These examples given above are merely illustrative and are not meant to be an exhaustive list of all possible designs, embodiments, applications, or modifications of the disclosure.

## Claims

1. An electronic smoking system comprising:
a first pack (200A) communicatively linked to a first electronic smoking device (100A) the first pack (200A) configured to hold and charge the first device (100A);
a second pack (200B) communicatively linked to a second electronic smoking device (100B),
the second pack (200B) configured to hold and charge the second device (100B);
a first computer (320A) communicatively linked to the first pack (200A) and to at least one communication channel; and
a second computer (320B) communicatively linked to the second pack (200B), the communication channel, and the first computer (320A);
**characterized in that** at least one of the first and second packs (200A, 200B) or the first and second computers (320A, 320B)) are configured to allow a first user of the first pack (200A) and a second user of the second pack (200B) to participate in a competition based on usage data received from the first computer (320A) and the second computer (320B) via a dedicated challenge application (914) on the first and second computer (320A, 320B); and
wherein the usage data comprises at least one of a maximum or minimum number of puffs taken per day on the first device (100A) and the second device (100B), respectively.

2. The system of claim 1, wherein at least one of the first pack or the second pack is directly communicatively linked to the communication channel.

3. The system of claim 1, wherein at least one of a social network, a vendor, a clinical monitoring program, or a data collection program is configured to match the first user of the first pack with the second user of the second pack according to the usage data.

4. The system of claim 1, wherein the at least one of the first computer or the second computer comprises an application, the application configured to receive data from at least one of the first pack or the second pack, respectively; and wherein the application is communicatively linked to the communication channel.

5. A method comprising:
receiving first data recorded or stored on a first pack (200A) communicatively linked to a first electronic smoking device (100A) and a first computer (320A), the first pack (200A) configured to hold and charge the first device (100A);
receiving second data recorded or stored on a second pack (200B) communicatively linked to a second electronic smoking device (100B) and a second computer (320B), the second pack (200B) configured to hold and charge the second device (100B); **characterized in** the steps of:
allowing a first user of the first pack (200A) or the first device (100A) to compete with a second user of the second pack (200B) or the second device (100B) based on the first data and second data via a dedicated challenge application (914) on the first and second computer (320A, 320B); and
wherein the first data and the second data comprise at least one of a maximum or minimum number of puffs taken per day on the first device (100A) and the second device (100B), respectively.

6. The method of claim 5, wherein at least one of the first data or the second data further comprise at least one of a duration of each puff taken by the first user or the second user, respectively; a flavor of smoke juice used in the first device or the second device, respectively; an amount of smoke juice in the first device or the second device, respectively; a temperature of vapor produced by the first device or the second device, respectively; a battery charge of the first device or the second device, respectively; a battery charge of the first pack or the second pack, respectively; a location of the first device or the second device, respectively; a location of the first pack or the second pack, respectively; and a location of the first user or the second user, respectively.

7. The method of claim 5, wherein allowing the first user to compete with the second user comprises providing at least one of a an application status update, an e-mail, a text message, a voicemail, a push notification, or a pop-up notification.

8. Machine-readable media comprising machine-readable instructions recorded thereon which, when executed on a first computer (320A) linked to a first pack (200A) and on a second computer (320B) linked to a second pack (200B) cause the first and second computer to execute the steps of:
receiving first data recorded or stored on a first pack (200A) communicatively linked to a first electronic smoking device (100A), the first pack (200A) configured to hold and charge the first device (100A);
receiving second data recorded or stored on a second pack (200B) communicatively linked to a second electronic smoking device (100B), the second pack (200B) configured to hold and charge the second device (100B); **characterized in**:
allowing a first user of the first pack (200A) or the first device (100A) to compete with a second user of the second pack (200B) or the second device (100B) based on the first data and second data via a dedicated challenge application (914) on the first and second computer (320A, 320B); and
wherein the first data and the second data comprise at least one of a maximum or minimum number of puffs taken per day on the first device (100A) and the second device (100B), respectively.

## Patentansprüche

1. Elektronisches Rauchsystem, umfassend:
eine erste Packung (200A), die mit einer ersten elektronischen Rauchvorrichtung (100A) kommunikativ verbunden ist, wobei die erste Packung (200A) so konfiguriert ist, dass sie die erste Vorrichtung (100A) enthält und auflädt;
eine zweite Packung (200B), die mit einer zweiten elektronischen Rauchvorrichtung (100B) kommunikativ verbunden ist, wobei die zweite Packung (200B) so konfiguriert ist, dass sie die zweite Vorrichtung (100B) enthält und auflädt;
einen ersten Computer (320A), der mit der ersten Packung (200A) und mit mindestens einem Kommunikationskanal kommunikativ verbunden ist; und
einen zweiten Computer (320B), der mit der zweiten Packung (200B), dem Kommunikationskanal und dem ersten Computer (320A) kommunikativ verbunden ist; **dadurch gekennzeichnet, dass**
mindestens eines aus der ersten und zweiten Packung (200A, 200B) oder dem ersten und zweiten Computer (320A, 320B) so konfiguriert ist, dass es einem ersten Benutzer der ersten Packung (200A) und einem zweiten Benutzer der zweiten Packung (200B) die Teilnahme an einem Wettbewerb auf der Grundlage von Nutzungsdaten, empfangen von dem ersten Computer (320A) und dem zweiten Computer (320B), über eine dedizierte Herausforderungsanwendung (914) auf dem ersten und zweiten Computer (320A, 320B) ermöglicht; und
wobei die Nutzungsdaten mindestens eine aus einer maximalen oder minimalen Anzahl von pro Tag an der ersten Vorrichtung (100A) bzw. der zweiten Vorrichtung (100B) getätigten Zügen umfassen.

2. System nach Anspruch 1, wobei mindestens eine aus der ersten Packung oder der zweiten Packung direkt mit dem Kommunikationskanal kommunikativ verbunden ist.

3. System nach Anspruch 1, wobei mindestens eines aus einem sozialen Netzwerk, einem Verkäufer, einem klinischen Überwachungsprogramm oder einem Datenerfassungsprogramm so konfiguriert ist, dass es den ersten Benutzer der ersten Packung dem zweiten Benutzer der zweiten Packung gemäß den Nutzungsdaten zuordnet.

4. System nach Anspruch 1, wobei der mindestens eine aus dem ersten Computer oder dem zweiten Computer eine Anwendung umfasst, wobei die Anwendung so konfiguriert ist, dass sie Daten von mindestens einer aus der ersten Packung bzw. der zweiten Packung empfängt; und wobei die Anwendung kommunikativ mit dem Kommunikationskanal verbunden ist.

5. Verfahren, umfassend:
Empfangen von ersten Daten, aufgezeichnet oder gespeichert in einer ersten Packung (200A), die mit einer ersten elektronischen Rauchvorrichtung (100A) und einem ersten Computer (320A) kommunikativ verbunden ist, wobei die erste Packung (200A) so konfiguriert ist, dass sie die erste Vorrichtung (100A) enthält und auflädt;
Empfangen von zweiten Daten, aufgezeichnet oder gespeichert in einer zweiten Packung (200B), die mit einer zweiten elektronischen Rauchvorrichtung (100B) und einem zweiten Computer (320B) kommunikativ verbunden ist, wobei die zweite Packung (200B) so konfiguriert ist, dass sie die zweite Vorrichtung (100B) enthält und auflädt;
**gekennzeichnet durch** die folgenden Schritte:
Ermöglichen, dass ein erster Benutzer der ersten Packung (200A) oder der ersten Vorrichtung (100A) mit einem zweiten Benutzer der zweiten Packung (200B) oder der zweiten Vorrichtung (100B) in einem Wettbewerb auf der Grundlage der ersten Daten und zweiten Daten über eine dedizierte Herausforderungsanwendung (914) auf dem ersten und zweiten Computer (320A, 320B) steht; und
wobei die ersten Daten und die zweiten Daten mindestens eine aus einer maximalen oder minimalen Anzahl von pro Tag an der ersten Vorrichtung (100A) bzw. der zweiten Vorrichtung (100B) getätigten Zügen umfassen.

6. Verfahren nach Anspruch 5, wobei mindestens eines aus den ersten Daten oder den zweiten Daten ferner mindestens eines aus einer Dauer jedes durch den ersten Benutzer bzw. den zweiten Benutzer getätigten Zuges; einem Geschmack von in der ersten Vorrichtung bzw. der zweiten Vorrichtung verwendetem Rauchsaft; einer Menge von Rauchsaft in der ersten Vorrichtung bzw. der zweiten Vorrichtung; einer Temperatur von durch die erste Vorrichtung bzw. die zweite Vorrichtung produziertem Dampf; einer Batterieladung der ersten Vorrichtung bzw. der zweiten Vorrichtung; einer Batterieladung der ersten Packung bzw. der zweiten Packung; einem Standort der ersten Vorrichtung bzw. der zweiten Vorrichtung; einem Standort der ersten Packung bzw. der zweiten Packung; und einem Standort des ersten Benutzers bzw. des zweiten Benutzers umfasst.

7. Verfahren nach Anspruch 5, wobei das Ermöglichen, dass der erste Benutzer mit dem zweiten Benutzer in einem Wettbewerb steht, das Bereitstellen von mindestens einem aus einer Anwendungsstatusaktualisierung, einer E-Mail, einer Textnachricht, einer Sprachnachricht, einer Push-Benachrichtigung oder einer Pop-up-Benachrichtigung umfasst.

8. Maschinenlesbare Medien, umfassend auf ihnen aufgezeichnete maschinenlesbare Befehle, die, wenn sie auf einem ersten Computer (320A), der mit einer ersten Packung (200A) verbunden ist, und auf einem zweiten Computer (320B), der mit einer zweiten Packung (200B) verbunden ist, ausgeführt werden, bewirken, dass der erste und zweite Computer die folgenden Schritte ausführen:
Empfangen von ersten Daten, aufgezeichnet oder gespeichert in einer ersten Packung (200A), die mit einer ersten elektronischen Rauchvorrichtung (100A) kommunikativ verbunden ist, wobei die erste Packung (200A) so konfiguriert ist, dass sie die erste Vorrichtung (100A) enthält und auflädt;
Empfangen von zweiten Daten, aufgezeichnet oder gespeichert in einer zweiten Packung (200B), die mit einer zweiten elektronischen Rauchvorrichtung (100B) kommunikativ verbunden ist, wobei die zweite Packung (200B) so konfiguriert ist, dass sie die zweite Vorrichtung (100B) enthält und auflädt;
**gekennzeichnet durch**:
Ermöglichen, dass ein erster Benutzer der ersten Packung (200A) oder der ersten Vorrichtung (100A) mit einem zweiten Benutzer der zweiten Packung (200B) oder der zweiten Vorrichtung (100B) in einem Wettbewerb auf der Grundlage der ersten Daten und zweiten Daten über eine dedizierte Herausforderungsanwendung (914) auf dem ersten und zweiten Computer (320A, 320B) steht; und
wobei die ersten Daten und die zweiten Daten mindestens eine aus einer maximalen oder minimalen Anzahl von pro Tag an der ersten Vorrichtung (100A) bzw. der zweiten Vorrichtung (100B) getätigten Zügen umfassen.

## Revendications

1. Système de cigarette électronique, comprenant :
un premier paquet (200A) en liaison de communication avec un premier dispositif de cigarette électronique (100A), ledit premier paquet (200A) étant prévu pour maintenir et charger le premier dispositif (100A) ;
un deuxième paquet (200B) en liaison de communication avec un deuxième dispositif de cigarette électronique (100B), le deuxième paquet (200B) étant prévu pour maintenir et charger le deuxième dispositif (100B) ;
un premier ordinateur (320A) en liaison de communication avec le premier paquet (200A) et avec au moins un canal de communication ; et
un deuxième ordinateur (320B) en liaison de communication avec le deuxième paquet (200B), le canal de communication et le premier ordinateur (320A) ;
**caractérisé**
**en ce que** au moins un parmi le premier et le deuxième paquet (200A, 200B) ou le premier et le deuxième ordinateurs (320A, 320B) sont prévus pour permettre à un premier utilisateur du premier paquet (200A) et à un deuxième utilisateur du deuxième paquet (200B) d'être mis en concurrence sur la base de données d'utilisation reçues du premier ordinateur (320A) et du deuxième ordinateur (320B) via une application de concurrence dédiée (914) sur le premier et le deuxième ordinateurs (320A, 320B) ; et où les données d'utilisation comprennent au moins un parmi un nombre maximum et un nombre minimum de bouffées inhalées quotidiennement respectivement sur le premier dispositif (100A) et sur le deuxième dispositif (100B).

2. Système selon la revendication 1, où au moins un élément parmi le premier paquet et le deuxième paquet sont en liaison de communication directe avec le canal de communication.

3. Système selon la revendication 1, où au moins un élément parmi un réseau social, un fournisseur, un programme de surveillance clinique ou un programme de collecte de données est prévu pour faire correspondre le premier utilisateur du premier paquet au deuxième utilisateur du deuxième paquet conformément aux données d'utilisation.

4. Système selon la revendication 1, où le au moins un parmi le premier ordinateur et le deuxième ordinateur comprennent une application, ladite application étant prévue pour recevoir au moins un parmi des données du premier paquet et du deuxième paquet ; et où ladite application est en liaison de communication avec le canal de communication.

5. Procédé, comprenant :
la réception de premières données enregistrées ou stockées sur un premier paquet (200A) en liaison de communication avec un premier dispositif de cigarette électronique (100A) et un premier ordinateur (320A), ledit premier paquet (200A) étant prévu pour maintenir et charger le premier dispositif (100A) ;
la réception de deuxièmes données enregistrées ou stockées sur un deuxième paquet (200B) en liaison de communication avec un deuxième dispositif de cigarette électronique (100B) et un deuxième ordinateur (320B), ledit deuxième paquet (200B) étant prévu pour maintenir et charger le deuxième dispositif (100B) ;
**caractérisé par** l'étape suivante :
permettre à un premier utilisateur du premier paquet (200A) ou du premier dispositif (100A) d'être mis en concurrence avec un deuxième utilisateur du deuxième paquet (200B) ou du deuxième dispositif (100B) sur la base des premières données et des deuxièmes données via une application de concurrence dédiée (914) sur le premier et le deuxième ordinateur (320A, 320B) ; et
où les premières données et les deuxièmes données comprennent au moins un parmi un nombre maximum ou un nombre minimum de bouffées inhalées quotidiennement respectivement sur le premier dispositif (100A) et sur le deuxième dispositif (100B).

6. Procédé selon la revendication 5, où les premières données et/ou les deuxièmes données comprennent en outre au moins un élément parmi une durée de chaque bouffée respectivement inhalée par le premier utilisateur ou the deuxième utilisateur, un arôme d'e-liquide respectivement utilisé dans le premier dispositif ou le deuxième dispositif, une quantité d'e-liquide respectivement présent dans le premier dispositif ou le deuxième dispositif, une température de vapeur respectivement produite par le premier dispositif ou le deuxième dispositif, une charge respective de batterie du premier dispositif ou du deuxième dispositif et une charge respective de batterie du premier paquet ou du deuxième paquet, une localisation respective du premier dispositif ou du deuxième dispositif, une localisation respective du premier paquet ou du deuxième paquet et une localisation respective du premier utilisateur ou du deuxième utilisateur.

7. Procédé selon la revendication 5, où la permission accordée au premier utilisateur d'être mis en concurrence avec le deuxième utilisateur comprend la transmission d'au moins un élément parmi une mise à jour de statut d'application, un message électronique, un message texte et un message, une notification push et une notification à affichage rapide.

8. Support lisible par machine comprenant des instructions enregistrées lisibles par machine, lesquelles, une fois exécutées sur un premier ordinateur (320A) relié à un premier paquet (200A) et sur un deuxième ordinateur (320B) relié à un deuxième paquet (200B), entraînent l'exécution des étapes suivantes par le premier et le deuxième ordinateurs :
réception de premières données enregistrées ou stockées sur un premier paquet (200A) en liaison de communication avec un premier dispositif de cigarette électronique (100A), ledit premier paquet (200A) étant prévu pour maintenir et charger le premier dispositif (100A) ;
réception de deuxièmes données enregistrées ou stockées sur un deuxième paquet (200B) en liaison de communication avec un deuxième dispositif de cigarette électronique (100B), ledit deuxième paquet (200B) étant prévu pour maintenir et charger le deuxième dispositif (100B) ; **caractérisé** :
**en ce qu'**il permet à un premier utilisateur du premier paquet (200A) ou du premier dispositif (100A) d'être mis en concurrence avec un deuxième utilisateur du deuxième paquet (200B) ou du deuxième dispositif (100B) sur la base des premières données et des deuxièmes données via une application de concurrence dédiée (914) sur le premier et le deuxième ordinateur (320A, 320B) ; et
où les premières données et les deuxièmes données comprennent au moins un parmi un nombre maximum ou un nombre minimum de bouffées inhalées quotidiennement respectivement sur le premier dispositif (100A) et sur le deuxième dispositif (100B).
